# EUROPEAN PATENT APPLICATION

(11) **EP 3 705 154 A1**
(43) Date of publication of application: **09.09.2020**
(21) Application number: 18873881.9
(22) Date of filing: 27.10.2018
(51) Int. Cl.: A61M 29/02

(54) **ATRIAL SEPTOSTOMY DEVICE, ATRIAL SEPTOSTOMY SYSTEM, OPERATING METHOD FOR SAME, AND OPENING-CREATION METHOD**

(30) Priority: 31.10.2017 CN 201711052922; 14.05.2018 CN 201810457374
(71) Applicant: Hangzhou Noya Medtech Co., Ltd, Zhejiang 310052 (CN)
(72) Inventor: WANG, Yongsheng, Hangzhou Zhejiang 310052 (CN); DONG, Yuanbo, Hangzhou Zhejiang 310052 (CN); LI, Jianmin, Hangzhou Zhejiang 310052 (CN); ZI, Zhenjun, Hangzhou Zhejiang 310052 (CN)
(74) Representative: Hamer, Christopher K.
(86) International application number: PCT/CN2018/112269
(87) International publication number: WO 2019/085841

(57) **Abstract**

An interatrial shunt device includes an opening main body. The opening main body includes an opening component for penetrating the atrial septum and expanding the atrial septal tissue to form an opening. An ablation mechanism that may damage the tissue activity at the opening of the atrial septum is disposed where the opening component contacts the atrial septal tissue. An interatrial shunt system includes the interatrial shunt device, a control mechanism of the interatrial shunt device, and an ablation power source.

## Description

### CROSS-REFERENCE TO RELATED APPLICATIONS

This application claims the priority and benefit of International Application No. PCT/CN2018/112269, filed on October 27, 2018 and published as WO 2019/085841, which claims the priority and benefit of Chinese Application CN 201711052922.7, filed on October 31, 2017, and Chinese Application CN 201810457374.4, filed on May 14, 2018. The contents of all afore-mentioned applications are hereby incorporated by reference in their entirety.

### TECHNICAL FIELD

The present disclosure relates to the field of medical instruments, and relates to an interatrial shunt device for percutaneous intervention, in particular to an interatrial shunt device, an interatrial shunt system, an operation method thereof and an opening method.

### BACKGROUND

Heart failure is a complex clinical syndrome in which ventricular filling or impaired ejection capacity is impaired by any abnormal cardiac structure or malfunction. The main clinical symptoms are dyspnea and fatigue (limited activity tolerance), and fluid retention (pulmonary congestion and peripheral edema). Heart failure is referred to as a serious and final stage of various heart diseases, and its incidence is high. It is one of the most important cardiovascular diseases today. According to the location of heart failure, it can be divided into left heart failure, right heart failure, and whole heart failure.

Heart failure is a serious disease with a high incidence and mortality. The incidence of heart failure in PR. China is 2-3%, and the number of incidences is more than twelve million. The main causes of heart failure include hypertension, coronary heart disease, myocardial infarction, heart valve disease, atrial fibrillation, and cardiomyopathy. Cardiovascular disease causes left ventricular damage, leading to pathological reconstruction of the left ventricle, resulting in cardiac dysfunction. Each successful treatment of a patient with a myocardial infarction brings a potential heart failure patient.

In the treatment, after optimizing the drug treatment, the patient's symptoms are still recurrent, and the current drug treatment has positive results almost only on patients with reduced ejection fraction. For patients who retains the ejection fraction, the effect is not ideal. Cardiac resynchronization therapy (CRT) is not suitable for all heart failure patients, and cardiac resynchronization pacing is not effective on more than 20% of patients. Left ventricular assist device (LVAD) surgery requires extracorporeal circulation trauma and has a high incidence of complications, also is expensive and difficult to obtain, and is not listed in China. Heart transplantation is the ultimate solution, but the source of the donor is very limited and expensive.

On the other hand, pulmonary arterial hypertension (PAH) is a group of diseases characterized by progressive increase in circulatory resistance of the pulmonary system. Its pathological changes include pulmonary vasoconstriction and reconstruction, abnormal proliferation of pulmonary vascular smooth muscle, endothelial cells, in situ thrombosis, etc., eventually leading to right heart failure and consequently death. At present, with the research on the pathogenesis of pulmonary hypertension, the treatment methods also keep emerging. The treatment plan for pulmonary hypertension should be individualized and systematic, and it cannot use a single drug to treat. The treatment methods include: general treatment, non-specific drug therapy, targeted drug therapy, NO inhalation therapy, gene therapy, intervention and surgical treatment. In the late stage of PAH patients, after the above comprehensive treatments, the effect is often not obvious, the survival rate is low, and the prognosis is very poor. At this time, surgical treatment methods such as atrial septal opening, lung transplantation, cardiopulmonary transplantation can be tried to save the patient's life, but this type of treatment has many factors such as high surgical risk, lack of donors, transplant rejection, and high cost of follow-up treatment.

Interatrial shunts refers to creating an opening at the patient's atrial septum to create a shunt of the left and right atriums, which can be used to treat pulmonary hypertension (right to left shunt) or left heart failure (left to right shunt), and has been clinically proven with its effectiveness.

Traditional interatrial shunts methods, such as balloon interatrial shunts, have a tendency for the myocardial tissue to rebound after creating the opening, and the opening will shrink or even close completely after a period of time. In order to solve the problem of shrinkage or even closure of the opening, an atrial septum opening stent is provided in the prior art. And an implant for atrial shunt can be separately disclosed, which is characterized by, after performing the puncturing of a percutaneous transluminal septum, an implant by skin delivery implants a shunt device at the atrial septum to maintain a smooth opening at the shunt opening.

Another interatrial shunts device includes a cutting device and a grasping device. When the interatrial shunts device is creating an opening at the tissue, the grasping device first positions and grasps a part of the tissue to be cut, then the cutting component of the cutting device performs a cutting to the part of the tissue grasped by the grasping device. The part of the tissue cut is taken out of the body by the grasping device to form an opening.

The above technique has the following drawbacks: an implant for atrial shunt, which leaves a device at the opening, is prone to thrombosis, or the instrument is detached, forming an embolism. In addition, due to endothelial grafting, the opening of the instrument can be blocked, and the passage closure loses the shunting effect. In addition, the cutting of the intracardiac tissue by mechanical or high-frequency electrosurgical during the operation has a higher risk, such as loosening during the operation of the intraoperative grasping device or during retrieval, which may cause the cut tissue to fall off and form an embolism. In addition, during the cutting process, the loosening of the grasping device is likely to cause damage to other myocardial tissue.

### SUMMARY

The technical problems to be solved by the present disclosure relate to, in contrast to the disadvantages of the prior art, providing a recoverable interatrial shunt device and its interatrial shunt system, which do not need cutting the tissues and are not easy to form embolisms.

The distal end and the proximal end of the present disclosure are relative to the operator. The closer end to the operator in the interatrial shunts device is the proximal end, and the end farther away from the operator is the distal end.

The present disclosure provides an interatrial shunt device, including an opening main body. The opening main body includes an opening component. The opening component penetrates through an atrial septum and expands the atrial septal tissue to create an atrial septal opening. An ablation mechanism capable of damaging tissue activity of the atrial septal opening is disposed where the opening component contacts the atrial septal tissue.

The interatrial shunt device may penetrate an opening on the atrial septum in advance, or may use the opening main body to puncture to form the opening.

The interatrial shunt device of the present disclosure is applied in a specific environment, that is, ablation is performed at an opening. Since the opening is different from a general cavity scenario (for example, an intravascular), there is a special positioning requirement. Therefore, an extension component connected with the opening main body is provided, for compensating or preventing a deviation of the ablation mechanism from the atrial septal opening.

The extension component is generally located on opposite sides or one side of the opening, and is positioned to abut against the atrial septum during use, or to extend the ablation mechanism. Under normal circumstances, the length of the ablation mechanism in the axial direction of the opening main body is substantially adapted to the thickness of the atrial septum, but if an deviation happens, the ablation effect is inevitably affected. The positional deviation of the ablation mechanism can be compensated by the auxiliary ablation of the extension component.

The ablation mechanism ablates an atrial septal opening tissue by any or a combination of heating, cooling, lighting, electrifying, gas, mechanical waves, electromagnetic waves, radioactive particles, and chemicals. The ablation mechanism can physically or chemically inactivate the protein denaturation of the atrial septal tissue cells in contact with it, thereby making the opening structure stable and difficult to close.

Further, the ablation mechanism increases or decreases a local temperature of the atrial septal tissue in contact with the ablation mechanism.

The ablation mechanism is a radio frequency ablation mechanism, and the radio frequency ablation mechanism is electrically conductive with an ablation power source during operation.

In the present disclosure, in the ablation process, ablation is performed by energizing the ablation mechanism to release energy to the opening site of the atrial septum. For the ablation power source, the control device including the ablation power source can adopt conventional methods such as radio frequency ablation used with an indifferent electrode.

The opening main body includes at least an opening component in a columnar structure in an axial direction.

The opening component of the opening main body may be formed integrally, or formed separately and mutually fixed. The opening component is used to expand the atrial septal tissue, and may use its own shapes and dimensions, or may adopt a radial expansion manner to accommodate different requirements of the dimensions of opening.

The opening main body includes at least an opening component in a tubular structure.

The opening component is provided with a compressed state in which a radial diameter is adjustable and an expanded state.

The opening main body is a balloon capable of radially contracting and expanding, an elastic bracket capable of radially contracting and expanding, or a combination of the balloon and the elastic bracket.

The opening main body as a whole can be used in various ways. When the balloon is combined with the elastic bracket, the balloon can be located wholly or partially inside the elastic bracket, or the balloon and the elastic bracket can be arranged side by side and connected to each other.

The opening main body is a balloon capable of radially contracting and expanding, an elastic bracket capable of radially contracting and expanding, or a combination of the balloon and the elastic bracket.

The opening component may be a part of the opening main body, that is, a section in the axial direction, or may be that the opening component is the opening main body.

The opening component is an elastic bracket capable of radially contracting and expanding in a tubular or annular structure formed by a wave structure, a mesh bracket, a rod bracket or a combination thereof.

The extension component of the interatrial shunt device is configured as:
after the opening main body penetrates through the atrial septum, the extension component is divided by the atrial septum into a first part of the extension component on one side of the atrial septum and a second part of the extension component on the other side of the atrial septum;
the ablation mechanism extends along an axial direction of the opening main body together with the opening component, when the opening main body penetrates through the atrial septum the ablation mechanism extends to one or two sides outside the atrial septal opening, a part of the ablation mechanism located outside the atrial septal opening being the first part of the extension component;
and the second part of the extension component is configured as:
the opening main body is connected with at least one positioning mechanism, when the opening main body penetrates through the atrial septum the positioning mechanism abuts against an outer periphery of the atrial septal opening on a corresponding side of the atrial septum.

When the extension component is disposed on both sides of the opening component, the same or different ablation manners may be used, and for compensation requirements, the parts of the extension component on both sides are separately controlled during the ablation.

The opening main body is connected with at least one positioning mechanism. The positioning mechanism can stably fix the opening component at the opening, to prevent the movement of the instruments caused by the pulsating of the heart and affecting the ablation effect.

In the interatrial shunts device, when the opening main body penetrates the atrial septum, the positioning mechanism is disposed on one or both sides of the atrial septum.

The positioning mechanism is disposed on one or both sides of the ablation mechanism.

In the interatrial shunt device, the positioning mechanism is provided with a positioning surface, a positioning line, or a positioning point abutting against the atrial septum, and the positioning surface is a plane, a cone, an arc, or a combination of the plane, the cone, and the arc.

In the interatrial shunt device, the positioning mechanism is coupled to a thrombosis capturing mechanism. The thrombosis capturing mechanism is a cage-shape structure.

In the interatrial shunt device, the positioning mechanism is provided with at least one radio-opaque marker.

In the interatrial shunt device, the ablation mechanism has a radio frequency ablation structure, and is electrically connected to an ablation power source during operation. The ablation mechanism is a metal exposed on an outer surface of the opening component or a metal electrode fixed on the outer surface of the opening component.

In the interatrial shunt device, a proximal end of the opening main body is provided with a retrieval department, and the retrieval department is provided with a connector for connecting with a control mechanism of the opening main body, the connector is electrically conductive with the ablation mechanism.

In the interatrial shunt device, the outer surfaces of the opening main body and the opening component are insulative except where the ablation mechanism contacts the atrial septal tissue.

In the interatrial shunt device, the outer surface being insulative means it is coated by an insulating coating.

In the interatrial shunt device, the ablation mechanism is provided with a connecting part for connecting a control mechanism of the opening main body, the connecting part being electrically conductive with the ablation mechanism.

In the interatrial shunt device, an insulator is disposed between the ablation mechanism and the opening component to prevent electrical conduction between the ablation mechanism and the opening component.

The ablation mechanism is a metal exposed on an outer surface of the opening component, and an insulator is disposed between the ablation mechanism and the opening component to prevent electrical conduction between the ablation mechanism and the opening component. The ablation mechanism is provided with a connecting part for connecting a control mechanism of the opening main body, the connecting part being electrically conductive with the ablation mechanism.

In the interatrial shunt device, the opening component is provided with an adjustment mechanism that adjusts the radial diameter of the opening component.

In the interatrial shunt device, the adjustment mechanism includes at least one control string, the control string simultaneously passing through different positions of the opening component circumferentially, a dimension of the opening component being adjusted by controlling a length of a string crossing the circumferential direction of the opening component.

In the interatrial shunt device, the adjustment mechanism includes at least two control strings, each of the at least two control strings respectively passing through a different position of the opening component in the circumferential direction and converging toward a center of the opening component into a bundle, and fixed to limit the radial diameter of the opening component.

The adjustment mechanism includes a fluid delivery device in communication with a balloon.

In the interatrial shunt device, the ablation mechanism is coupled to a temperature sensor in contact with the atrial septal tissue, the temperature sensor being electrically coupled to the ablation power source.

In the interatrial shunt device, the ablation mechanism is provided with at least one radio-opaque marker.

An interatrial shunt system, including the interatrial shunt device, the control mechanism of the shunt device, and the ablative power source, the ablation power source is electrically coupled to the ablation mechanism via the control mechanism of the shunt device.

In the interatrial shunt system, the shunt device is integrally fixedly connected to or detachably fixedly connected to the control mechanism of the shunt device.

In the interatrial shunt system, the control mechanism of the shunt device includes a pusher of the shunt device. The shunt device is integrally fixedly connected to or detachably fixedly connected to the control mechanism of the shunt device by the pusher. The pusher may push the shunt device to the opening of the atrial septal tissue.

In the interatrial shunt system, the control mechanism of the shunt device includes a pusher of the shunt device, and an outer sheath assembly that carries the pusher and the shunt device. The outer sheath assembly may load the radially expandable shunt device in the compressed state in the sheath, and deliver to the opening of the atrial septal tissue in an intervening manner to ablate the opening.

In the interatrial shunt system, the control mechanism of the shunt device includes a pusher of the shunt device, an outer sheath assembly that carries the pusher and the shunt device, and a sheath core assembly inserted into the pusher. A delivery channel may be established by the sheath core assembly to more conveniently and accurately deliver the shunt device to the opening site of the atrial septal tissue.

In the interatrial shunt system, the control mechanism of the shunt device includes an adjustment assembly that controls the radial diameter of the opening component. The adjustment assembly may adjust the radial diameter of the opening component according to requirements, to adjust the size of the opening on the atrial septum.

The adjustment assembly includes a traction string and a control tube. The radial diameter of the opening may be adjusted by the adjustment mechanism by connecting the traction string with the diameter adjustment mechanism of the shunt device.

Alternatively, the adjustment assembly includes a fluid delivery device, particularly for a balloon-type opening device, the size of the expanded balloon may be adjusted so as to adjust the size of the opening on the atrial septum.

A method of operating an interatrial shunt system, including:
puncturing an atrial septum;
using the interatrial shunt device according to any of claims 1-28 and delivering the interatrial shunt device to an atrial septal tissue opening by the control mechanism of the shunt device;
releasing the interatrial shunt device and forming a shunt channel on the atrial septal tissue by the opening component of the interatrial shunt device;
determining and confirming that a tissue at the atrial septal opening is completely conformed to the ablation mechanism of the interatrial shunt device, and launching the ablation;
after the ablation stops, withdrawing the interatrial shunt device.

In the above-discussed method of operating the interatrial shunt system, in the step of delivering the interatrial shunt device to the atrial septal tissue opening by the control mechanism of the shunt device, the control mechanism of the shunt device includes an outer sheath, and the interatrial shunt device is loaded into the outer sheath and then transported to the atrial septal tissue opening.

In the above-discussed method of operating the interatrial shunt system, in the step of releasing the interatrial shunt device and forming a shunt channel on the atrial septal tissue by the opening component of the interatrial shunt device, the interatrial shunt device is radially expandable to further expand the tissue.

In the above-discussed method of operating the interatrial shunt system, the interatrial shunt device includes a positioning mechanism, the positioning mechanism stabilizing the shunt device at the atrial septal opening before the launching the ablation.

In the above-discussed method of operating the interatrial shunt system, in the step of determining and confirming that the tissue at the atrial septal opening is completely conformed to the ablation mechanism of the interatrial shunt device, determining whether the shunt device is conformed to the atrial septal tissue by monitoring an impedance (or resistance) change.

A method of forming an atrial septum opening, including: puncturing an atrial septum to form an atrial septal opening. An interatrial shunt device is delivered into the atrial septum opening, and a tissue at the atrial septum opening is ablated to cause the tissue lose activity.

The details about the structures of the interatrial shunt device and the method of forming an atrial septum opening, may be combined with the descriptions in the method of operating the interatrial shunt system.

In the method of forming an atrial septum opening, the tissue at the opening is an intraoral tissue.

In the method of forming an atrial septum opening, the ablation manner is radio frequency ablation.

In the method of forming an atrial septum opening, the interatrial shunt device includes an opening main body. The opening main body includes an opening component. The opening component penetrates through an atrial septum and expands an atrial septal tissue to form an atrial septal opening. An ablation mechanism capable of damaging tissue activity of the atrial septal opening is disposed where the opening component contacts the atrial septal tissue. The ablation mechanism is electrically connected to an ablation power source during operation.

In the method of forming an atrial septum opening, before electrifying the ablation mechanism of the interatrial shunt device, one must confirm whether the contacting position of the ablation mechanism with the atrial septum is appropriate; whether the ablation mechanism of the interatrial shunt device is appropriately in contact with the atrial septum is determined by monitoring an impedance (or resistance) change.

In the method of forming an atrial septum opening, an extension component connected with the opening main body is provided, for compensating or preventing a deviation of the ablation mechanism from the atrial septal opening.

The extension component of the interatrial shunt device is configured as:
after the opening main body penetrates through the atrial septum, the extension component is divided by the atrial septum into a first part of the extension component on one side of the atrial septum and a second part of the extension component on the other side of the atrial septum;
the ablation mechanism extends along an axial direction of the opening main body together with the opening component, when the opening main body penetrates through the atrial septum the ablation mechanism extends to one or two sides outside the atrial septal opening, a part of the ablation mechanism located outside the atrial septal opening being the first part of the extension component;
and the second part of the extension component is configured as:
the opening main body is connected with at least one positioning mechanism, when the opening main body penetrates through the atrial septum the positioning mechanism abuts against an outer periphery of the atrial septal opening on a corresponding side of the atrial septum.
The interatrial shunt device is connected with at least one positioning mechanism. Before electrifying the ablation mechanism of the interatrial shunt device, the contact between the ablation mechanism and the atrial septum may be adjusted and maintained by the positioning mechanism, so as to make sure the contact site between the ablation mechanism and the atrial septum is appropriate before electrifying.

The opening main body is connected with at least one positioning mechanism. The positioning mechanism is disposed on one or both sides of the atrial septum

The positioning mechanism of the interatrial shunt device has a diameter larger than that of the ablation mechanism on one side or both sides.

In the method of forming an atrial septum opening, the interatrial shunt device includes an opening main body. The opening main body is in a columnar structure, and a part of the columnar structure in the axial direction is the opening component. The opening component has a constant radial diameter or is radially expandable.

In the method of forming an atrial septum opening, the interatrial shunt device includes an opening main body. The opening main body is in a tubular structure, and a part of the tubular structure in the axial direction is the opening component. The opening component has a radial diameter in a compressed state and an expanded state.

In the method of forming an atrial septum opening, the opening main body is a balloon capable of radially contracting and expanding, an elastic bracket capable of radially contracting and expanding, or a combination of the balloon and the elastic bracket.

The opening component is an elastic bracket capable of radially contracting and expanding in a tubular or annular structure formed by a wave structure, a mesh bracket, a rod bracket or a combination thereof.

In the method of forming an atrial septum opening, the interatrial shunt device is delivered by the control mechanism of the interatrial shunt device to the opening of the atrial septum. Optionally, the control mechanism is fixedly connected or detachably fixedly connected to the interatrial shunt device.

In the method of forming an atrial septum opening, after the ablation is completed, the control mechanism withdraws the interatrial shunt device.

In the method of forming an atrial septum opening, the control mechanism includes an outer sheath. After the ablation is completed, the mechanism withdraws the interatrial shunt device into the sheath, and retracts overall.

In the method of forming an atrial septum opening, after the ablation is completed, the control mechanism is released from the interatrial shunt device, the control mechanism retracts, and the interatrial shunt device remains at the opening of the atrial septum tissue. The interatrial shunt device is provided with a channel in the axial direction.

In the interatrial shunt device and the interatrial shunt system, the opening component is used to expand the atrial septum after the puncturing, and the ablation mechanism is used to ablate the atrial septum tissue, to avoid the risk of embolization by cutting the tissue. And the instruments after the opening is formed can be recycled to avoid problems such as the instruments falling off.

The operation method of the present disclosure can avoid the need of cutting tissue in the prior art during surgery, which causes the risk of embolization, and the whole operation process is smoother and simpler, and the device after the surgery can be retrieved, thereby avoiding problems such as falling off of the instrument.

### BRIEF DESCRIPTION OF THE DRAWINGS

To illustrate the technical solutions according to the embodiments of the present disclosure or in the prior art more clearly, the accompanying drawings for describing the embodiments or the prior art are introduced briefly in the following. Apparently, the accompanying drawings in the following description are only some embodiments of the present disclosure, and persons of ordinary skill in the art can derive other drawings from the accompanying drawings without creative efforts.
FIG. 1a is a schematic structural diagram of a first embodiment of the present disclosure.
FIG. 1b is a schematic structural diagram of an implementation of the first embodiment of the present disclosure.
FIG. 2 is a schematic structural diagram of an implementation of the first embodiment of the present disclosure.
FIG. 3 is a schematic structural diagram of an implementation of the first embodiment of the present disclosure.
FIG. 4 is a schematic structural diagram of a second embodiment of the present disclosure.
FIG. 5 is a cross-sectional view along the line A-A of FIG. 4.
FIG. 6 is a cross-sectional view along the line B-B of FIG. 4.
FIG. 7 is a schematic structural diagram of a third embodiment of the present disclosure.
FIG. 8 is a cross-sectional view along the line C-C of FIG. 7.
FIG. 9 is a partially enlarged perspective view at E of FIG. 7.
FIGS. 10-11 are exploded perspective views of an opening component of the third embodiment of the present disclosure.
FIG. 12 is a schematic structural diagram of a fourth embodiment of the present disclosure.
FIG. 13 is a schematic structural diagram of a fifth embodiment of the present disclosure.
FIGS. 14-15 are schematic structural diagrams of a metal electrode of the fifth embodiment of the present disclosure.
FIG. 16 is a schematic structural diagram of a sixth embodiment of the present disclosure.
FIG. 17 is a cross-sectional view along the line A-A of FIG. 16.
FIG. 18 is a cross-sectional view along the line B-B of FIG. 16.
FIG. 19 is a cross-sectional view along the line C-C of FIG. 16.
FIG. 20 is a schematic structural diagram of an implementation of the sixth embodiment of the present disclosure.
FIG. 21 is a schematic structural diagram of a first implementation of the interatrial shunt system in accordance with a seventh embodiment of the present disclosure.
FIG. 22 is a schematic structural diagram of a second implementation of the interatrial shunt system in accordance with the seventh embodiment of the present disclosure.
FIG. 23 is a schematic structural diagram of the second implementation of the interatrial shunt system when the shunt device is constrained in the sheath in accordance with the seventh embodiment of the present disclosure.
FIG. 24 is a schematic structural diagram of a third implementation of the interatrial shunt system in accordance with the seventh embodiment of the present disclosure.
FIG. 25 is a partially enlarged perspective view at D of FIG. 24.
FIG. 26 is a cross-sectional view along the line F-F of FIG. 24.
FIG. 27 is a cross-sectional view along the line H-H of FIG. 24.
FIGS. 28-29 are schematic structural diagrams of the interatrial shunt device of an eighth embodiment of the present disclosure.
FIG. 30 is a schematic structural diagrams of the control mechanism of the interatrial shunt device of the eighth embodiment of the present disclosure.

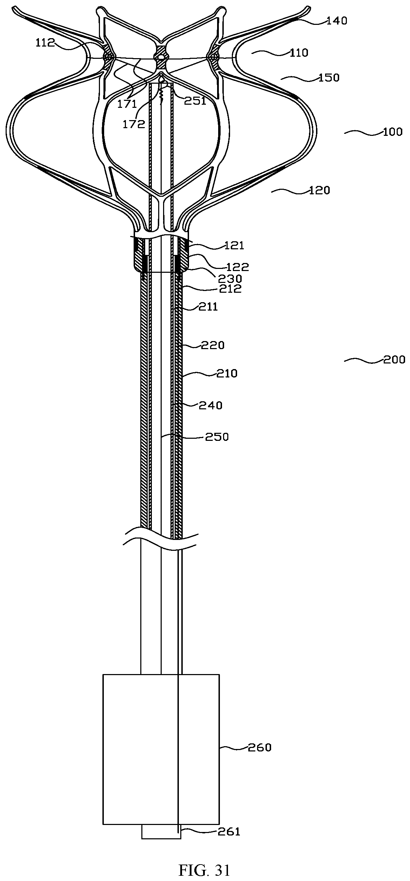

FIG. 31 is a schematic structural diagram of the interatrial shunt system of the eighth embodiment of the present disclosure.
FIGS. 32-35 are schematic flowcharts of the implantation operation of the interatrial shunt system of the eighth embodiment of the present disclosure.
FIGS. 36 and 36a are schematic structural diagrams of a ninth embodiment of the present disclosure.
FIGS. 37a-37e are schematic diagrams illustrating the opening component at different stages in accordance with a tenth embodiment of the present disclosure.
FIGS. 38a-38f are schematic diagrams illustrating the opening component at different stages in accordance with an eleventh embodiment of the present disclosure.

### DETAILED DESCRIPTION OF THE EMBODIMENTS

The present disclosure will be described in details in combination with the accompanying drawings and embodiments such that the purpose, technical solution and advantages of the present disclosure will be more apparent. It should be understood that the particular embodiments are described for the purpose of illustrating as opposed to restricting the present disclosure.

### First Embodiment:

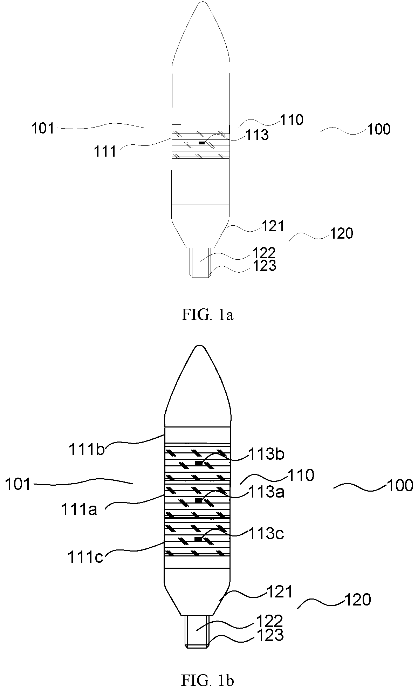

As shown in FIG. 1a, the basic structure of an interatrial shunt device 100 includes an opening main body 101 that is generally in a columnar structure. The opening main body 101 is provided with an opening component 110 for penetrating through the atrial septum and expanding the atrial septal tissue. In this embodiment, the opening component 110 of the columnar structure is disposed in the middle of the opening main body 101. The radial diameter of the opening component is fixed, and the opening component 110 is provided with an ablation mechanism 111 that is electrically connected to the ablation power source and is disposed at least on the outer surface in a circumferential direction.

As shown in FIG. 1b, in another implementation, with respect to FIG. 1a, the ablation mechanism 111a is in the middle, and an extension component 111b and an extension component 111c are on both sides, respectively. If the ablation mechanism 111a is slightly deviated in use, by way of compensation of the extension component 111b and the extension component 111c, the ablation may be applied to the atrial septum.

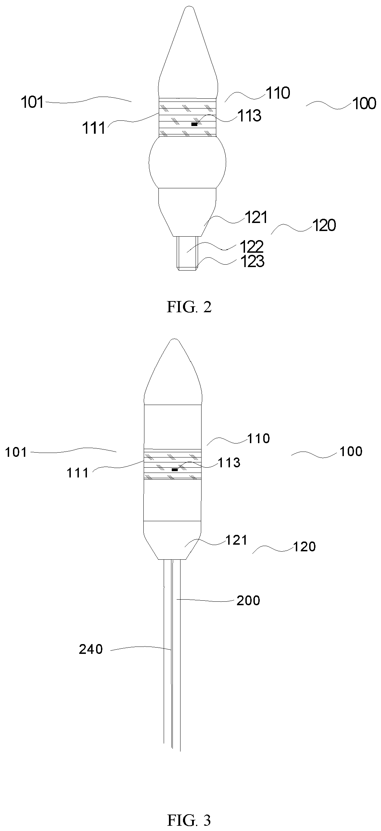

As shown in FIG. 2, the shape of the opening main body 101 may be various. For example, the opening main body 101 may be a concave or/and convex curved surface, a cylindrical shape, an elliptical cylinder shape or a combination thereof. The curved shape forms a closed curved surface structure in the circumferential direction, and the positions of the convex and concave portions can be set as needed. The convex or concave structure can be separately formed, or the convex or concave structure can be combined and disposed on the same opening main body 101. The convex structure is, for example, a disk shape, a spherical shape, or the like, and the concave structure is, for example, a waist drum shape. In this embodiment, a cylindrical structure is adopted, and a straight cylindrical shape of the opening main body 101 smoothly transits to form an integral cylindrical structure. The axial length of the opening component is set according to actual needs, and generally matches the thickness of the atrial septal tissue.

The ablation mechanism 111 is disposed on the opening component 110. The ablation mechanism 111 is a metal electrode attached to the outer surface of the opening component 110. The shape of the ablation mechanism 111 may be a separate sheet shape, a network shape, a rod shape, or the like according to the shape of the opening component 110, and a plurality of intervals may be arranged around the opening component 110. The ablation mechanism 111 may also be an ablation mechanism 111 that is provided with a continuous or intermittent annular structure around the opening component 110.

Since the ablation mechanism 111 is electrically conductive for ablation of part of the tissue of the opening component 110, it can be electrically conductive only to the corresponding opening tissue, and cannot affect other parts of the heart. Therefore, an insulation member must be disposed between the ablation mechanism 111 and the opening component 110 to avoid conduction between the ablation mechanism 111 and the opening component 110. Alternatively, other opening components 110 except the ablation mechanism 111 and the opening main body 101 are insulated at least on the outer surface in contact with blood. In this embodiment, except for the ablation mechanism, the other parts of the opening main body are insulated at least when the outer surface of the atrial septal tissue is in contact.

As shown in FIG. 2, the convex diameter-enlarging structure under the ablation mechanism 111 prevents the ablation mechanism from deviating from the opening formed on the atrial septal tissue by the opening component 101, can be used for positioning, and is treated as one form of the extension component.

The ablation mechanism 111 may extend upwardly and may be regarded as one form of the extension component, i.e., a combination of ablation compensation and positioning.

As shown in FIG. 1a, in order to realize the retrieval of the interatrial shunt device 100, a proximal end of the opening main body 101 is connected to the retrieval department 120, and the retrieval department 120 is provided with a connector for connecting with the delivery system. In this embodiment, the proximal end of the retrieval department 120 shrinks to form a retrieval port 121. A connector is provided in the retrieval port 121. The connector may be various depending on the retrieval method. The connector of this embodiment employs a nut 122 of an internal thread 123 disposed at the retrieval port 121, and is screwed to the nut 122 of the internal thread 123 by an external thread of the push rod of the pushing mechanism.

As shown in FIG. 3, in another embodiment, the retrieval port 121 is fixedly coupled to the pusher.

As shown in FIG. 3, since the ablation mechanism 111 needs to be electrically connected to the ablation power source, the power conduction can be performed in various ways, a direct wire connection or by a metal structure of the opening main body 101. In this embodiment, the metal structure of the opening main body 101 is adopted. An insulating layer is coated on the surfaces of the opening main body 101, the opening component 110, and the retrieval department 120. Since the connector is electrically connected to the ablation mechanism 111, the nut 122 serving as a connector is not coated with an insulating layer, i.e., the internal thread 123 in the nut 122 is not coated with an insulating layer, and is screwed to the pusher in the delivery system. The ablation mechanism 111 and the ablation power source are electrically connected through the conductive delivery system.

As shown in FIG. 1a, the ablation mechanism 111 is provided with at least one radiopaque marker 113, i.e., at least one radiopaque marker hole is opened in the ablation mechanism 111. The radiopaque material is filled in the radiopaque marker hole to form a radiopaque marker113, and the radiopaque material may be selected from gold, platinum, platinum-tungsten, palladium, platinum-ruthenium, osmium, iridium, or alloys or composites of these metals. This embodiment uses a golden radiopaque marker 113. The filling method may be mechanical deformation such as inlaying, welding, bonding, and the like. The radiopaque marker113 is used to display the position of the ablation mechanism 111 during surgery, for accurately placing the ablation mechanism 111 at the atrial septal tissue opening.

As shown in FIG. 1b, the ablation mechanism 111a, the extension component 111b, and the extension component 111c are each provided with a radiopaque marker, which is respectively a radiopaque marker113a, a radiopaque marker113b, and a radiopaque marker113c in the drawing.

The operation method of the interatrial shunt system of this embodiment is:
1) puncturing through the femoral vein, performing the atrial septum puncture, and retaining the guide wire in the left atrium;
2) pushing the interatrial shunt device along the guide wire through the control mechanism of the interatrial shunt device to penetrate and extend the atrial septum;
3) under the observation of ultrasound or DSC, adjusting the position of the shunt device, and causing the annular ablation mechanism 111 of the shunt device (a single annular electrode or an arrangement of a plurality of electrodes) to contact the atrial septal tissue;
4) by methods like impedance changes (when the ablation mechanism is conformed to the atrial septal tissue, the impedance is significantly increased), confirming that the annular ablation mechanism of the opening catheter is in contact with the atrial septal tissue, and discharging and ablating;
5) at the end of the ablation, withdrawing the instruments from the body.

### Second Embodiment:

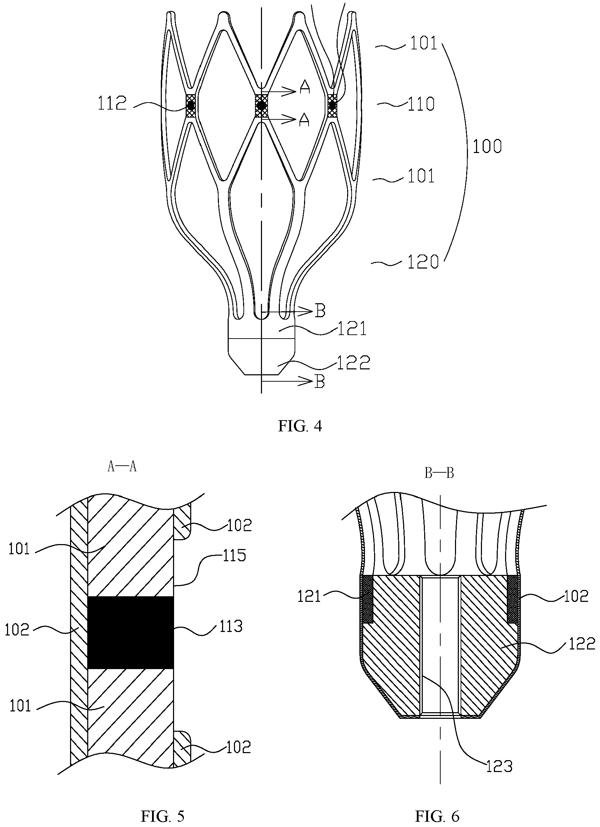

As shown in FIGS. 4-6, the basic structure of an interatrial shunt device100 includes an opening main body 101 that is radially expandable. The opening main body 101 is provided with an opening component 110 for penetrating through the atrial septum and radially expanding the atrial septal tissue. In this embodiment, the opening component 110 is disposed at a distal end of the opening main body 101. The opening component 110 is provided with an ablation mechanism 111 electrically connected to the ablation power source at least on the outer surface in a circumferential direction. The ablation mechanism 111 is electrically conductive at least on a surface in contact with the atrial septum. An insulating member is provided between the ablation mechanism 111 and the opening component 110 to avoid conduction between the two, or the outer surface of the opening component 110 and the opening main body 101 except for the ablation mechanism 111 is insulative.

The opening main body 101 is an elastic bracket that is capable of radially contracting and expanding. In this embodiment, the opening main body 101 is a nickel alloy bracket, and can be made by cutting a nickel alloy tube, or by weaving nickel alloy wires. The degree of density of the mesh structure of the opening main body 101 is set as needed. In this embodiment, a diamond-shaped structural unit is used to form a continuous circumferential arrangement. The overall shape of the opening main body 101 may be a straight shape, a disk shape, a taper shape or the like, and is not limited herein. When the opening main body 101 is delivered through the sheath, the diameter can be contracted to a smaller state for facilitating the delivery in the sheath; when released in the heart, it can automatically expand to the desired shape and size, and can produce some radial supporting functions for the tissue in contact.

The main function of the opening component 110 is to expand the atrial septal tissue with radial expansion. The opening component 110 is disposed on the opening main body 101, particularly in the distal or middle parts of the opening main body. The opening component 110 may have many structures, as long as it may be disposed in a circumferential direction of the opening main body and can uniformly expand the atrial septal tissue. Particularly, the opening component 110 is a tubular structure or an annular structure formed by a wave stent, a mesh stent, a rod stent or a combination thereof.

The tubular structure can be understood as extending a distance in the axial direction. For example, the axial dimension is greater than or equal to the outer diameter of the tubular structure. With respect to the tubular structure, the axial dimension of the annular structure is slightly smaller, and generally smaller than the outer diameter of the annular structure.

The mesh stent has a distinct warp and weft interlaced structure, or has a repeating cell block structure, which can be either woven or cut, and the warp and weft interlaced portions can be relatively slid or fixed to each other.

The wave stent has a multi-turn circular waveform structure including peaks, troughs, and poles. Circumferentially adjacent poles are connected at the proximal ends to form peaks, and are connected at the distal ends to form troughs; the axially adjacent two-turn waveform structures can by connected by films, or a plurality of films are fixedly attached to a tubular film.

The rod stent has a plurality of support rods extending axially, and the support rods form a tubular structure. The support rods may be connected by a high molecular polymer film connection, or the support rods may be fixedly connected to the tubular film.

In this embodiment, the opening component 110 is a wave-shaped annular structure connected to the diamond structure of the opening main body 101 to form one or more layers of a grid structure. In consistence with the opening main body 101, the opening component 110 also requires radial contraction and is included in the sheath.

The shape of the opening component 110 may be various. For example, the opening component 110 may be a concave or/and convex curved surface, a cylindrical shape, an elliptical cylinder shape or a combination thereof. The curved shape forms a closed curved surface structure in the circumferential direction, and the positions of the convex and concave portions can be set as needed, and the convex or concave structure can be separately formed, or the convex or concave structure can be combined on the same opening component 110. The convex structure is, for example, a disk shape, a spherical shape, or the like, and the concave structure is, for example, a waist drum shape. In this embodiment, a cylindrical structure is adopted, and a straight cylindrical shape of the opening main body 101 smoothly transits to form an integral cylindrical structure. The axial length of the opening component is set according to actual needs, and generally matches the thickness of the atrial septal tissue.

The ablation mechanism 111 is disposed on the opening component 110, and is divided into two cases according to the structure of the ablation mechanism 111: in one case the ablation mechanism 111 is an exposed conductive metal member, and in the other case the ablation mechanism 111 is a metal electrode attached to and fixed on an outer surface of the opening component 110. In this embodiment, the ablation mechanism 111 is an exposed conductive metal member, and the conductive metal member may be separately disposed and fixed to the opening component 110, or may be a part of the opening component 110, or the ablation mechanism 111 and the opening component 110 may be formed integrally. When the ablation mechanism 111 is separately disposed, the ablation mechanism 111 is made of metal and fixed to the opening component 110 by inlaying or pasting. When the ablation mechanism 111 is a part of the opening component 110, the ablation mechanism 111 is directly made of a metal material of the opening component 110. Also, the ablation mechanism 111 may be an exposed metal on the outer surface of the opening component 110, and is directly used as the ablation mechanism 111. The ablation mechanism 111 as an exposed conductive metal member means that the ablation mechanism 111 is directly made of metal. The shape of the ablation mechanism 111 can be in a shape of a sheet, a network, or a rod, which cooperate with the shape of the opening component 110 and are mutually independent, and a plurality is arranged around the opening component 110 at intervals in a circumference. The ablation mechanism 111 may also be an ablation mechanism 111 that is provided with a continuous or intermittent annular structure around the opening component 110 in a circumference. One round of the annular structure is a structure that can be contracted toward the center or a soft-flexed structure, convenient for being received in the sheath.

Since the ablation mechanism 111 is electrically conductive for ablation of part of the tissue of the opening component 110, it can only be electrically connected to the corresponding opening tissue, and cannot affect other parts of the heart. Therefore, an insulation member must be disposed between the ablation mechanism 111 and the opening component 110 to avoid conduction between the ablation mechanism 111 and the opening component 110, or, other opening component 110 and the opening main body 101 except the ablation mechanism 111 are insulated at least on the outer surface in contact with blood. In this embodiment, the opening component 110 in the trough top structure in the wave structure is directly used as the ablation mechanism 111. As shown in FIG. 5, on the surface of the opening component 110, except that the outer surface 115 of the opening component 110 facing towards the atrial septal tissue is a bare metal, the other outer surface of the opening component 110 is completely insulative, i.e., adopting an insulating coating 102 of Parylene. The outer surface insulation means that the surface is coated with an insulating coating 102.

As shown in FIG. 4 and FIG. 6, in order to achieve retrieval of the interatrial shunt device 100, the proximal end of the opening main body 101 is connected to a retrieval department 120, and the retrieval department 120 is provided with a connector for connecting to the delivery system. In this embodiment, the proximal end of the retrieval department 120 is contracted to form a retrieval port 121, and the retrieval port 121 has a connector. The connector may be various according to the retrieval method. In this embodiment, the connector is a nut 122 of an internal thread 123 in the retrieval port 121, and is screwed to the nut 122 of the internal thread 123 through the external thread of the push rod of the pushing mechanism.

As shown in FIG. 6, since the ablation mechanism 111 needs to be electrically connected to the ablation power source, the power conduction can be performed in various ways. A direct wire connection may be used or a metal structure of the opening main body 101 can be conductive. In this embodiment, the metal structure of the opening main body 101 is conductive, and an insulating layer is coated on the surface of the opening main body 101, the opening component 110, and the retrieval department 120. Since the connector is electrically connected to the ablation mechanism 111, the nut 122 as a connector is not coated with an insulating layer, i.e., the internal thread 123 in the nut 122 is not coated with an insulating layer, and is screwed to the pusher in the delivery system, and the ablation mechanism 111 and the ablation power source are electrically connected through the conductive delivery system.

As shown in FIG. 2, the ablation mechanism 111 is provided with at least one radiopaque marker 113, i.e., at least one radiopaque marker hole is disposed in the ablation mechanism 111. The developing material is filled in the radiopaque marker hole to form a radiopaque marker 113, and the developing material may be selected from gold, platinum, platinum-tungsten, palladium, platinum-ruthenium, osmium, iridium, or alloys or composites of these metals. This embodiment uses a golden radiopaque marker 113. The filling method may be mechanical deformation such as inlaying, welding, bonding, etc. The radiopaque marker 113 is used to display the position of the ablation mechanism 111 during surgery, for accurately placing the ablation mechanism 111 at the atrial septal tissue opening.

The operation method of the interatrial shunt system of this embodiment is:
1) puncturing the atrial septum with a puncture mechanism; after the puncturing, sending a guide wire into the upper left pulmonary vein, and withdrawing the puncture set; pushing the expander and sheath along the guide wire into the left atrium, and withdrawing the guide wire and the expander.
2) choosing an interatrial shunt device 100 of an appropriate size; making the pusher pass through the proximal end of the loader, and connecting the retrieval port 121 of the retrieval department 120 at the proximal end of the interatrial shunt device 100 to the distal end of the pusher; withdrawing the pusher to move the interatrial shunt device 100 into the loader.
3) connecting the distal end of the loader to the proximal end of the sheath; pushing forward the pusher to drive the interatrial shunt device 100 to the distal end of the sheath; observing and arranging the radiopaque marker 113 in the ablation mechanism 111 to be disposed in the atrial septal tissue; then slowly pushing the pusher or withdrawing back the sheath; ensuring that the radiopaque marker is located in the atrial septal tissue during the whole process, so that the opening component 110 of the interatrial shunt device 100 is fully opened and the atrial septal tissue at the opening is expanded to form a specific size shunt channel; and determining the size of the opening by ultrasound or DSC.
4) after confirming that the tissue at the opening is completely conformed to the ablation mechanism 111, connecting the proximal end of the pusher to the RF power source (the ablation power source), and setting the heating parameters (such as power 20-80W, duration 10-50S), then launching the heating.
5) after the heating is stopped, arranging the instruments to be received in the sheath and withdrawn from the body, and measuring the diameter of the opening against the expectation.

### Third Embodiment:

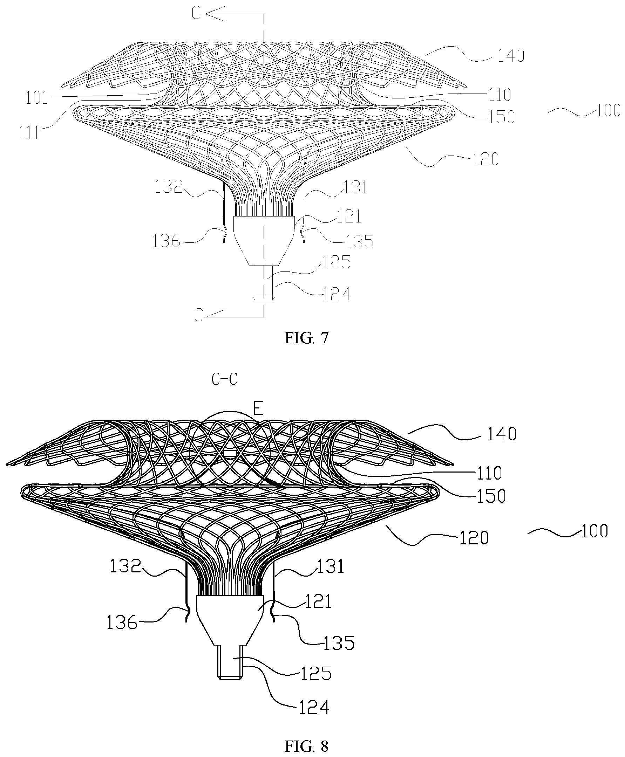

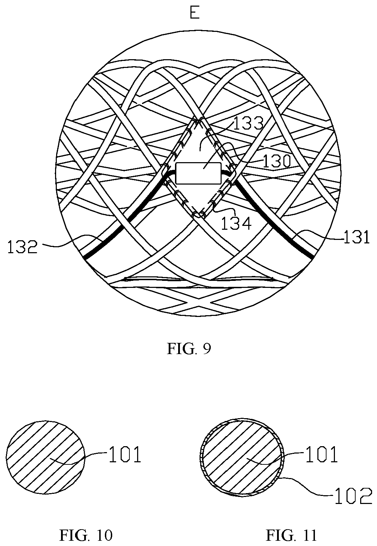

As shown in FIGS. 7-9, the present embodiment is improved on the basis of the second embodiment. The interatrial shunt device 100 includes a radially expandable opening main body 101. The middle part of the opening main body 101 is a tubular and radially expandable opening component 110. The opening component 110 is provided with an ablation mechanism 111 electrically connected to the ablation power source at least on the outer surface in a circumferential direction. The opening main body 101 is an integral structure woven by wires, and has a shape that is a waist drum shape or a frustum shape formed by a curved surface of the busbar recess.

In this embodiment, a positioning mechanism is added based on the second embodiment. The opening main body 101 is coupled to at least one positioning mechanism, and the proximal end and/or the distal end of the opening main body 101 has at least one positioning mechanism. After the implantation, the positioning mechanism is located on a side wall of the atrial septum or on both side walls of the atrial septum respectively. The positioning mechanism is used to fix the positioning of the interatrial shunt device 100 to keep it stable.

The positioning mechanism may be treated as one of the forms of the extension component, avoiding the ablation mechanism from deviating from the opening formed by the opening component 101 on the atrial septal tissue. The positioning mechanism is provided with a positioning surface, a positioning line, or a positioning point abutting against the wall of the atrial septum, and provides clamping or pressing by the positioning surface, the positioning line, or the positioning point. The positioning surface is a plane, a cone surface, a curved surface, or a combination thereof. The positioning line is a linear contact formed by the positioning mechanism and the wall of the atrial septum, and may be a straight line, a curve, or the like. The positioning point is at least a point contact formed by the positioning mechanism and the wall of the atrial septum. The positioning mechanism forming the positioning surface, the positioning line, and the positioning point may not have a defined shape and structure. As shown in FIGS. 7-9, in this embodiment, a positioning mechanism is formed by adopting a structure conforming to the opening main body 101 and the opening component 110, and two positioning mechanisms are respectively connected to the distal end and the proximal end of the opening main body 101, i.e., a left atrial positioning mechanism 140 and a right atrial positioning mechanism150, respectively. The left atrial positioning mechanism 140 is a tapered surface, and the tapered tip faces the left side to form an annularly-distributed positioning point. The right atrial positioning mechanism 150 is a planar structure forming a positioning surface.

As shown in FIGS. 7-9 and 10-11, a conical retrieval department 120 is connected to the outer edge of the right atrial positioning mechanism 150, and the cone tip of the retrieval department 120 forms a retrieval port 121 toward the right. The cone tip forming retrieval port 121 is provided with a metal screw 125 as a connector. Except for the outer surface of the ablation mechanism 111 and the externally threaded surface 124 of the metal screw 125, the remaining surfaces are plated with a Parylene insulating coating 102. The metal screw 125 is screwed to the pusher of the delivery system.

Further, a structural difference from the second embodiment is that the ablation mechanism 111 is connected to a temperature sensor in contact with the atrial septal tissue, and the temperature sensor is electrically connected to the ablation power source. As shown in FIGS. 7-9, specifically, on the opening component 110, a miniature thermistor 130 is disposed as a temperature sensor, and two ends of the thermistor 130 are soldered with two mutually insulated metal wires 131, 132. The micro thermistor 130 is wrapped inside two PI films 133 having good insulating properties. The two films 133 are integrally fused by soldering around the thermistor 130 and the thermistor 130 is completely encapsulated. The PI films 133 are stitched together with the opening component 110 by the suture 134. The wires 131 and 132 extend from the PI film package through the fusion zone, and are respectively coupled with elastic connectors 135 and 136 to be electrically conductive with the temperature detecting system of the ablation power source.

The remaining structure of this embodiment is the same as that of the second embodiment, and details are not described herein again.

The operation method of the interatrial shunt system of this embodiment is:
1) puncturing the atrial septum with a puncture mechanism; after the puncturing, sending a guide wire into the upper left pulmonary vein, and pushing the sheath along the guide wire into the left atrium; withdrawing the guide wire and the expander.
2) choosing an interatrial shunt device 100 of an appropriate size; arranging the pusher pass through the proximal end of the loader; connecting the retrieval port 121 of the retrieval department 120 at the proximal end of the interatrial shunt device 100 to the distal end of the pusher; and withdrawing the pusher to make the interatrial shunt device 100 received in the loader.
3) connecting the distal end of the loader to the proximal end of the sheath; pushing forward the pusher to drive the interatrial shunt device 100 to the distal end of the sheath; then slowly pushing the pusher forward, or withdrawing the sheath, ensuring that the distal end of the sheath is in the left atrium, making the left atrial positioning mechanism of the interatrial shunt device 100 fully opened; and determining whether the left atrial positioning mechanism is fully opened by ultrasound or DSC.
4) then making sure that there is no relative motion between the instruments and pulling the sheath back to make the left atrial positioning mechanism conformed to the atrial septum; holding the opening component and the pusher still, and withdrawing the sheath so that the opening component 110 and the right atrial positioning mechanism are fully open and the right atrial positioning mechanism is conformed to the atrial septum; observing by DSC the positions of the atrial septum and the ablation mechanism 111 to determine whether they are completely fitted.
5) after confirming that the tissue at the opening is completely fitted with the ablation mechanism 111, connecting the proximal end of the pusher to the RF power supply (the ablation power source), and setting the heating mode to temperature control mode, and selecting parameters (such as temperature 50-80 degrees, duration 10-50 seconds), then starting the heating.
6) after the heating is stopped, the instruments can be retrieved to the sheath and removed from the body, and the diameter of the opening is measured against the expectation.

### Fourth Embodiment:

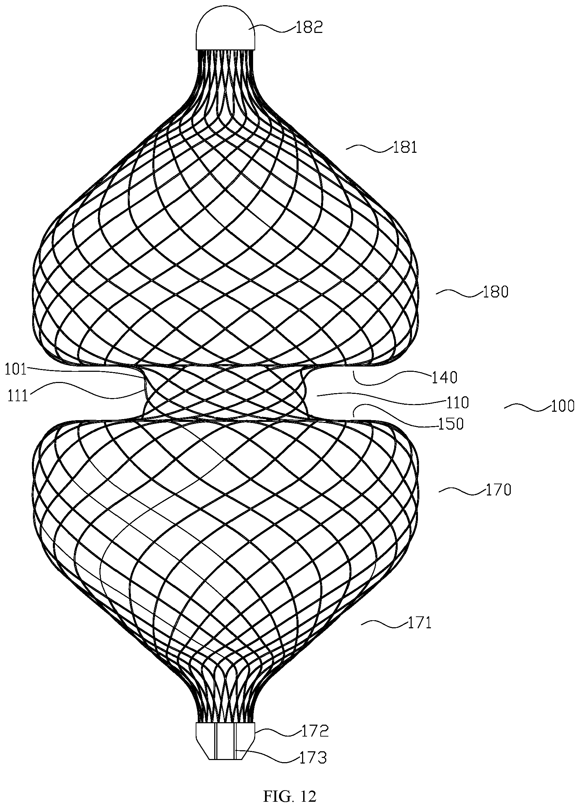

This embodiment is an improvement on the basis of the first, second, and third embodiments. As shown in FIG. 12, the interatrial shunt device 100 includes a tubular and radially expandable opening main body 101 and an opening component 110. The opening component 110 is provided with an ablation mechanism 111 electrically connected to the ablation power source at least on the outer surface in a circumferential direction.

The opening main body 101 and the opening component 110 are an integral structure woven by wires, and their shapes are cylindrical. Also, this embodiment adds a positioning mechanism to the second embodiment. The opening main body 101 is connected with two positioning mechanisms, and the positioning mechanisms are respectively located on the two side walls of the atrial septum after implantation. In this embodiment, the positioning mechanism is formed by adopting the structure conforming to the opening main body 101 and the opening component 110. At the distal end of the opening component 110 and the proximal end of the opening main body 101, two positioning mechanisms are respectively connected, i.e., a left atrial positioning mechanism 140 and a right atrial positioning mechanism150, respectively. The left atrial positioning mechanism 140 and the right atrial positioning mechanism 150 are planar flanges. The flat flange shape refers to the planar contact with the sidewalls of the atrial septum sidewall.

In addition to the above structure, in the present embodiment, the positioning mechanism is connected to a thrombosis capturing mechanism. The thrombosis capturing mechanism is a cage-shape structure, and the thrombosis capturing mechanism adopts a woven structure, which can be woven integrally together with the opening main body 101 and the opening component 110 and the positioning mechanism, or, a thrombosis capturing mechanism can be separately disposed then formed into an overall structure by welding. The structures of the thrombosis capturing mechanism, the opening main body 101, and the opening component 110 may be the same or different, i.e., the size of the mesh to be woven, the diameter of the wire, and the like may be the same or different.

As shown in FIG. 12, a cylindrical left atrial thrombosis capturing cage 180 extending towards the distal end is connected to the outer edge of the left atrial positioning mechanism 140, and they are integrally formed. The left atrial thrombosis capturing cage 180 is closed at the distal end, and the distal end closing surface 181 is a conical cone. A cylindrical right atrial thrombosis capturing cage 170 extending towards the proximal end is connected to the outer edge of the right atrial positioning mechanism 150, and they are integrally formed. The right atrial thrombosis capturing cage 170 is closed at the proximal end, and the proximal end closing surface 171 is a conical cone with the conical tip facing the proximal end and connecting the metal nut 172. The metal nut 172 acts as the retrieval department and the connector.

Since the structure of the ablation mechanism 111 is the same as those of the second and the third embodiments, the structure of the insulating coating 102 is also the same. Except for the outer surface 115 of the ablation mechanism 111 and the internally threaded surface 173 of the metal nut 172, the remaining surfaces are plated with a polytetrafluoroethylene (PTFE) insulating coating 102.

The remaining structure of this embodiment is the same as those of the second and the third embodiments, and details are not described herein again.

When the interatrial shunt device 100 in this embodiment is used, the thrombosis capturing cages in the two atrium chambers are deployed, and the three-dimensional space region near the heating region corresponding to the ablation mechanism 111 is covered to prevent an embolus formed by blood via heating into the blood circulation, thereby preventing embolism. In the same manner as the second and the third embodiments, the interatrial shunt device 100 in this embodiment needs to be used in combination with a loader, a sheath, an expander, an electrically conductive pusher, an ablation power source, a power supply connection line, a neutral electrode plate, and the like.

The interatrial shunt device 100 of this embodiment has no temperature detecting devices, and therefore is the same as the second embodiment when the interatrial shunt device 100 is connected to the pusher and the heating mode is selected. The remaining steps are basically the same as in the third embodiment, and the main difference is that after the interatrial shunt device 100 is pushed out of the sheath, there must be observation and arrangement that the thrombosis capturing cage is fully expanded, to ensure that the thrombosis capturing cage can catch the embolus formed by blood via heating, so as to prevent it from entering the blood circulation system, achieving the purpose of preventing embolism.

### Fifth Embodiment:

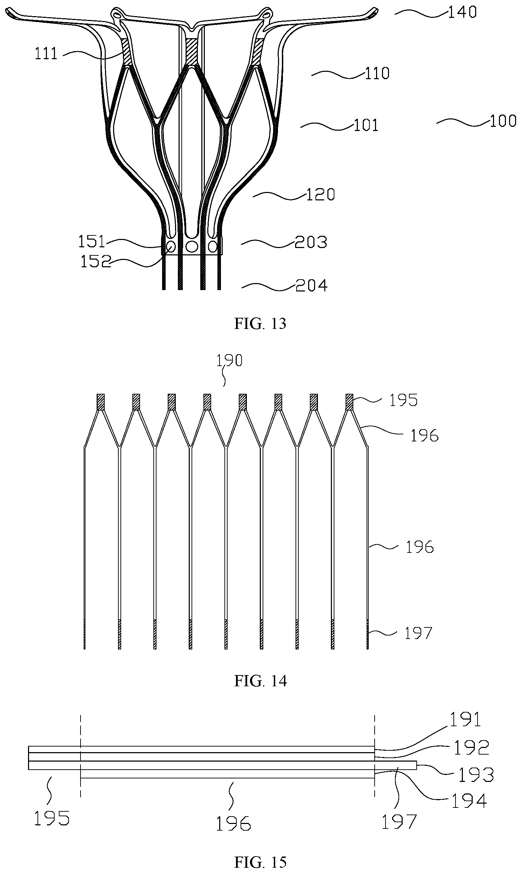

As shown in FIGS. 13-15, this embodiment is a modification of the embodiments first through fourth. The interatrial shunt device 100 includes a radially expandable opening main body 101, and the distal end of the opening main body 101 is a tubular and radially expandable opening component 110. The opening component 110 is provided with an ablation mechanism111 electrically connected to the ablation power source at least on the outer surface in a circumferential direction. The opening main body 101 and the opening component 110 are both a strut structure, and are formed by a plurality of struts cross-connecting, and have a cylindrical or elliptical cylindrical shape. In this embodiment, the positioning mechanism is formed by adopting the structure conforming to the opening component 110 itself. As shown in FIG. 13, the left atrial positioning mechanism 140 is a planar structure to form a positioning surface.

The difference in this embodiment is that the ablation mechanism 111 uses a metal electrode. As shown in FIG. 13, the metal electrode adopts a metal electrode. As shown in FIG. 15, the metal electrode 190 has a multilayer structure overall, and includes an adhesive 191, a PI substrate 192, an electrode copper layer 193, and a PI coating 194 from the inside to the outside.

As shown in FIG. 14, the metal electrode 190 has a multi-stage structure from the distal end to the proximal end, and includes an electrode portion 195, a guide wire portion 196, and a connecting portion 197. The electrode portion 195 is on the outer circumference of the opening component 110, and the guide wire portion 196 extends along the outer wall of the opening component 110 towards the proximal end till the connecting portion 197. The electrode portion 195 is composed of a glue layer 191, a PI substrate 192, and an electrode copper layer 193; the guide wire portion 196 is composed of a glue layer 191, a PI substrate 192, an electrode copper layer 193, and a PI coating 194; the connecting portion 197 is formed by an electrode copper layer 193. It can be seen from the drawing that there is no PI coating 194 on the outside of the electrode portion 195, and the electrode copper layer 193 is exposed to be in contact with the atrial septal tissue for ablation.

An insulator is disposed between the metal electrode 190 and the opening component 110 to prevent conduction between the two, or the opening component 110 where the metal electrode 190 is fitted is at least surface insulated. The two methods can both be used. The insulator can be an insulating sheet, an insulating coating, an insulating sleeve, and the like. In this embodiment, the surface of the nickel-titanium alloy stent 101 of the interatrial shunt device 100 is plated with the insulating coating 102 of PI to form an insulator to be insulated from the metal electrode 190.

As shown in FIG. 13, at the distal end of the opening component 110 and the proximal end of the opening main body 101, a positioning mechanism and a retrieval department 120 are respectively connected, specifically, a planar flange-shaped left atrial positioning mechanism 140 and a conical retrieval department 120 are respectively connected. The proximal end of the retrieval department 120 is converged to form a connecting port 151 as the connector. The connecting port 151 is a tubular structure, and eight fixing holes 152 are evenly distributed in the circumferential direction. The fixing holes 152 can serve as a joint with the PE protection tube 220 to improve the connecting strength with the PE protection tube 220.

The rest of the structure of the embodiment is the same as those of the embodiments second through fourth, and details are not described herein again.

### Sixth Embodiment:

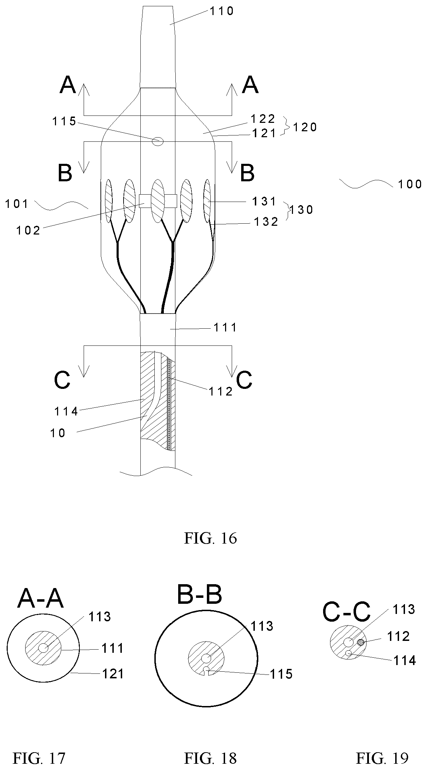

As shown in FIG. 16, a trans-catheter interventional interatrial shunt device includes a catheter opening main body 110, and a radially expandable balloon 120 fixed to the distal end of the catheter opening main body 110. The balloon 120 is provided for passing through the atrial septum and radially expanding the opening component 101 that expands the atrial septal tissue. The opening component 101 is provided with an electrode assembly 130 electrically connected to the ablation power source and the control mechanism at least on the outer surface of the balloon 120 in a circumferential direction. The catheter opening main body 110 is provided with a guide wire cavity 113 through which both ends are penetrated and a filling cavity 114 for filling the balloon 120 in the axial direction.

As shown in FIG. 16, the catheter opening main body 110 is used for implementing the support and delivery functions of the balloon 120, and is a tubular structure. The internal cavity is provided therein. According to different functions, the catheter opening main body 110 is at least provided with a guide wire cavity 113 through which both ends are penetrated and a filling chamber 114 for filling the balloon 120 in the axial direction. The guide wire cavity 113 is used for wearing the guide wire 10, and the filling cavity 114 is used for filling the balloon 120 with liquid or gas. In this embodiment, a guide wire cavity 113 is disposed in the catheter opening main body 110. The guide wire cavity 113 extends from the center of the distal end surface of the balloon 120 to the proximal end, and is bent toward the outer wall of the catheter at a position close to the proximal end of the balloon 120 and completely penetrates the wall of the catheter to form a through cavity used to place the guide wire 10.

As shown in FIGS. 17-19, a filling cavity 114 is disposed in the catheter opening main body 110. The filling cavity 114 is connected at the proximal end to the cavity of the joint at the proximal end of the catheter opening main body 110 through the sidewall hole, and extends towards the distal end along the catheter opening main body 110, and communicates only with the balloon cavity 122 of the balloon 120 through the sidewall hole 115 disposed on the distal end of the catheter opening main body 110, and fills and pressurizes the balloon 120 by the balloon cavity 122 of the balloon 120, making it inflated.

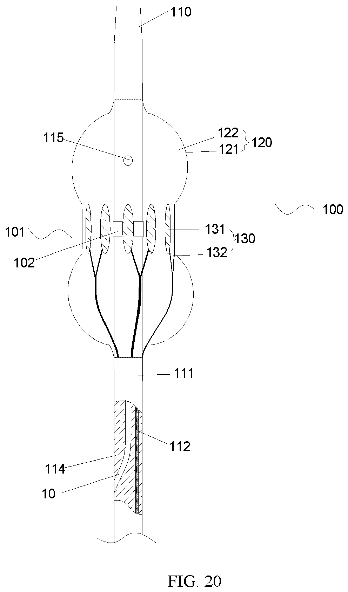

The balloon 120 is disposed on the distal end of the catheter opening main body 110, and there are various implementations for the number and arrangement of the balloons 120. In this embodiment, there is only one balloon 120, fixed to the distal end of the catheter opening main body 110. The balloon 120 may select a compliant balloon or a non-compliant balloon, and the shape may be spherical, cylindrical, figure-eight, tapered or their combinations. The shape referred to herein refers to the shape of the balloon 120 after it has been filled. In this embodiment, as shown in FIG. 16, the balloon 120 used is a non-compliant balloon, and the balloon 120 is cylindrical after being filled. When the balloon rides across the atrial septal tissue and is flushed, the balloon wall 121 provides functions of supporting and expanding to the atrial septal tissue such that the opening size is equal to or less than the post-filling diameter of the non-compliant balloon 120. As shown in FIG. 20, the balloon 120 is provided with a waist portion having a smaller diameter. The diameter of the balloon 120 gradually increases from the waist to the proximal end and/or the distal end, respectively, or at least one of the sides of the waist of the balloon 120 is provided with a positioning mechanism having a diameter larger than the diameter of the waist. In this embodiment, the balloon 120 having an outer contour of figure-eight is selected, i.e., in the middle portion of the axis of the balloon 120, there is a thin waist shape. The balloon 120 used is a non-compliant balloon. After the balloon 120 is filled, when the balloon 120 rides over the atrial septal tissue and is filled, the balloon wall 121 supports and expands the atrial septal tissue so that the opening size is equal to or approximately equal to the waist diameter of the non-compliance balloon.

The opening component 101 is disposed at a position near the center of the axis or near the center of the outer surface of the balloon 120. The opening component 101 is part of balloon 120. When the balloon rides across the atrial septal tissue, the position of the opening component 101 is placed in the puncturing position of the atrial septal tissue for expanding the atrial septal tissue.

As shown in FIG. 16, the electrode assembly 130 includes a connecting line 132 and an electrode 131. The electrode 131 is fixed on the outer surface of the balloon 120 in a circumferential direction, and is electrically connected to the ablation power source and the control mechanism through the connecting line 132. The electrode 131 disposed in the opening component 101 is a flexible electrode, and the electrode 131 is disposed at intervals in a plurality on the outer surface of the balloon 120 in a circumferential direction to form at least one electrode group, and all the electrodes 131 in each electrode group are connected to the same connecting line 132 or connected to a plurality of different connecting lines 132. Optionally, the plurality of electrodes 131 of the same electrode group are connected to the same connecting line 132. Disposing a plurality of electrode groups means grouping up the plurality of electrodes 131 on the outer surface of the balloon 120 in a circumferential direction. After grouping, each group controls the electrical connection respectively, and some or all of the electrode groups may be selected to be electrified, or the sequence of electrifying may be selected.

The electrode 131 is a monopolar ablation electrode or a bipolar ablation electrode. In this embodiment, twelve electrodes 131 are evenly distributed in the circumferential direction of the balloon 120 of the opening component 101, and the electrodes 131 are respectively parallel to the central axis of the balloon 120. The shape of the electrode 131 may be a circular or elliptical shape, a strip shape, a rod shape or the like. In this embodiment, the shape is elliptical, and its surface is a metal material having good electrical conductivity, such as copper, silver, gold. The distal end of the connecting line 132 is fixed to the proximal end of the electrode 131, and penetrates into the catheter opening main body 110 at the proximal end of the balloon 120 and is connected with the connector disposed at the proximal end of the catheter opening main body 110. In this embodiment, the connecting line 132 includes two parts: the first part is the first connecting line 132a, and the second part is the second connecting line 132b. That is, each of the two electrodes 131 forms a group of electrodes, and the two electrodes 131 of each electrode group are connected at the proximal end of the electrodes 131 to the same first connecting line 132a that is elongated, whose surface is completely insulated, and attached to the balloon wall 121. The proximal end of the first connecting line 132a extends towards the proximal end along the surface of the balloon 120, and is introduced into the catheter opening main body wall 111 at the connecting point between the proximal end of the balloon 120 and the catheter opening main body 110, and is connected by welding to the front end of the second connecting line 132b disposed in the opening main body 111. At the proximal end of the catheter opening main body 110, a connector for connecting the ablation power source is disposed, and the rear end of the second connecting line 132b in the catheter opening main body wall 111 is welded to the connector.

Further, in this embodiment, two balloons 120 are optionally disposed, and the two balloons 120 are nested with each other to form a double-layer balloon 120. The inner layer balloon 120 of the double-layer balloon 120 is a non-compliant balloon, and the outer layer balloon 120 is a compliant balloon. An electrode assembly 130 is disposed on the outer wall of the outer layer balloon 120.

The ablation current flows from the ablation power source, through the connector, the second connecting line 132b in the opening main body wall 111, the electrode 131, the first connecting line 132a, the electrode 131, the tissue (and blood), the inert electrode, and flows back to the ablation power source, to form an ablation circuit.

The catheter opening main body 110 is further provided with a developing member 102 for displaying a position during surgery. In the present embodiment, a developing ring as a developing member 102 is disposed at a position of the catheter opening main body 110 at the same cross section as the center of the opening component 101. Before the opening, according to the display of the developing member 102, the position of the balloon 120 is adjusted so that the developing member is just located in the atrial septal tissue, so that the electrode 131 is just attached to the atrial septal tissue which is expanded, thereby achieving accurate ablation of the opening.

Since the impedance of the circuit at the metal conductor is small, the conversion of electrical energy to heat is not significant. There is a higher impedance when conducting in tissue, and electrical energy is easily converted into heat. Particularly, there is the largest current density where the atrial septal tissue contacts the electrode 131, and the temperature rise in this region is very obvious, and can be as high as 60-90 °C. Since the current density decreases rapidly as the distance between the tissue and the electrode 131 increases, the heat generation by the resistor in the tissue about 1∼2 mm outside the contact surface is small (but there is still energy loss, just the current density is small and the heat is not obvious). The heat is transferred to a small area (5 mm) around the electrode 131 mainly by the heat transfer effect. Due to the impedance heating and heat transfer effect of the current, the atrial septal tissue around the electrode 131 is subject to heat and irreversible damage during the conduction process, and the atrial septal tissue wrapped around the balloon opening component 101 is necrotic and loses most of its elasticity, thereby making the size of the opening controllable.

### Seventh Embodiment:

This embodiment is a structure that is implemented in cooperation with the above-described embodiments first through sixth.

This embodiment is an interatrial shunt system including the interatrial shunt device 100, the interatrial shunt device control system 600, and the ablation power source of the embodiments first through sixth. The ablation power source is electrically connected to the ablation mechanism 111 of the interatrial shunt device 100. The interatrial shunt device 100, the interatrial shunt device control system, and the ablation power source are the basic structures of the interatrial shunt system. The ablation power source includes two parts, the ablation power source and the control device, and the ablation power source is used for power supply, and the control device is used for ablation control.

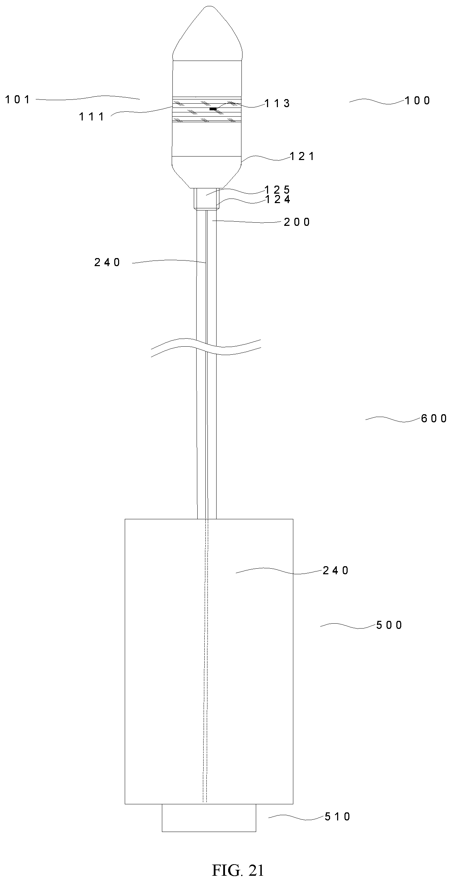

On the basis of the above structures, as shown in FIG. 21, an interatrial shunt system includes the interatrial shunt device 100, the interatrial shunt device control mechanism 600, and the ablation power source (not shown). The ablation mechanism 111 of the interatrial shunt device 100 by the control mechanism 600 is electrically connected to the ablation power source. The interatrial shunt device control system 600 includes a guide wire 240, a pusher 200, and a control handle 500. The pusher 200 is detachably connected or integrally fixedly connected with the interatrial shunt device 100. The ablation mechanism 111 is electrically connected to the guide wire 240.

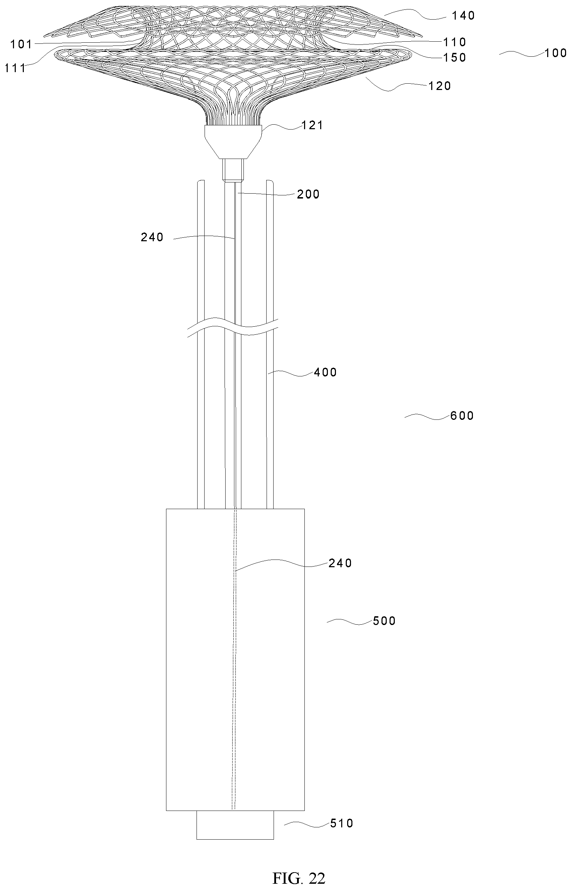

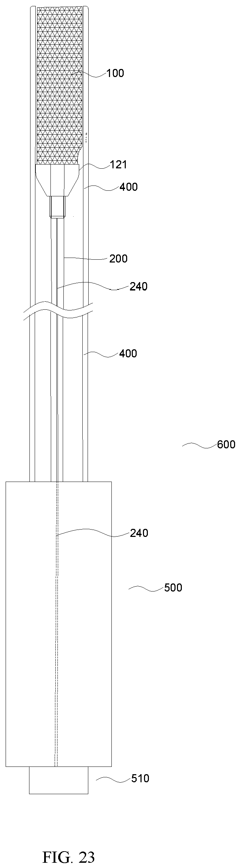

On the basis of the above structures, as shown in FIG. 22, an interatrial shunt system includes the above-mentioned interatrial shunt device 100, the interatrial shunt device control mechanism 600, and the ablation power source (not shown). The ablation mechanism 111 of the interatrial shunt device 100 by the control mechanism 600 is electrically connected to the ablation power source. The interatrial shunt device control system 600 includes a guide wire 240, a pusher 200, a control handle 500 and an outer sheath assembly 400. The pusher 200 is detachably connected or integrally fixedly connected to the interatrial shunt device 100. The guide wire 240 is electrically connected to the ablation mechanism 111. The interatrial shunt device 100 and the pusher 200 are wrapped by the outer sheath assembly. As shown in FIG. 23, the radially expandable interatrial shunt device 100 can be loaded into the outer sheath in a compressed state and transported to the opening of the atrial septal tissue by intervention.

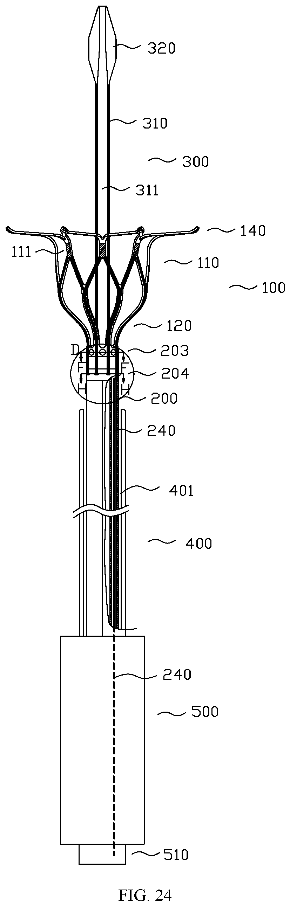

On the basis of the above structures, as shown in FIG. 24, an interatrial shunt system includes the above interatrial shunt device 100, the interatrial shunt device control mechanism 600, and the ablation power source (not shown). The ablation mechanism 111 of the interatrial shunt device 100 by the control mechanism 600 is electrically connected to the ablation power source. The interatrial shunt device control system 600 includes a guide wire 240, a pusher 200, a control handle 500 and an outer sheath assembly 400. The pusher 200 is detachably connected or integrally fixedly connected to the interatrial shunt device 100. The guide wire 240 is electrically connected to the ablation mechanism 111. The interatrial shunt device 100 and the pusher 200 are wrapped by an outer sheath assembly.

The ablation power source and its control mechanism are electrically connected to the ablation mechanism 111. The pusher is detachably fixedly coupled to the interatrial shunt device 100. The sheath mechanism includes a sheath tube and a sheath core that are fitted to each other. The proximal end of the pusher and the sheath mechanism is connected to a control handle 500. The interatrial shunt device 100 is radially contracted and received in the sheath tube. The sheath core assembly can be inserted into the guide wire, which can be positioned and released integrally, and the operation is more convenient.

The following is a detailed description of the interatrial shunt device of the fifth embodiment:

The interatrial shunt device 100 is the same as the fifth embodiment, and details are not described herein again.

As shown in FIGS. 13 and 24-27, the pusher 200 selects a dual cavity tube 210 having a cavity 201 and a cavity 202. The mechanical connection region 203 between the distal end of the pusher 200 and the proximal end of the interatrial shunt device 100 is connected by a heat fusion method. The connection method is to sleeve connecting port 151 at the proximal end of the interatrial shunt device 100 on the distal end of the pusher 200, and to sleeve the PE protection tube 220 on the tubular connection port 151 of the interatrial shunt device 100 to form a mechanical connection zone 203. At the same time, the guide wire portion 195 of the metal electrode 190 pass between the tubular connection port 151 and the PE protection tube 220.

As shown in FIG. 24, below the mechanical connection region 203 of the pusher 200, an electrical connection region 204 is provided. The electrical connection region 204 includes a metallic connecting ring 230. The connecting part 197 of the metal electrode 190 and the connecting ring 230 are electrically connected by soldering.

The metal electrode, as shown in the fifth embodiment, includes from the inside to outside (in a radial direction) an adhesive 191, a PI substrate 192, an electrode copper layer 193, and a PI coating 194. The outer surface of the opening main body 101 is coated with an insulating coating 102. The metal electrode 190 is fixed on the outer surface of the opening main body 101, and the insulating coating 102 is disposed between the metal electrode 190 and the opening component 110 to avoid electrical conduction between the two.

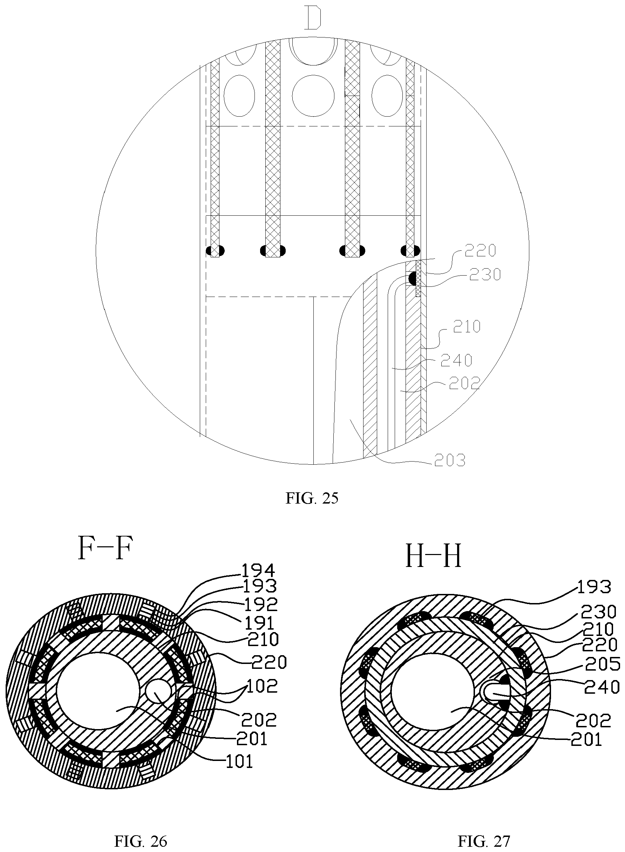

Referring to FIG. 27 and FIG. 25, a guide wire 240 is disposed in the cavity 202 of the pusher 200. The front end of the guide wire 240 passes through the tube wall 205 at the connecting ring 230 and is electrically connected to the connecting ring 230 by soldering. The PE protection tube 220 extends backwardly from the front end of the pusher 200 until it covers a length behind the connecting ring 230. In the electrical connection zone 204, all materials are also fused together, and the welding is completely melted inside the material, thereby ensuring the safety and reliability of the electrical connection.

The sheath 400 includes a sheath cavity 401 with a pusher 200 located within the sheath lumen 401. The sheath core 300 is located in the cavity 201 of the pusher 200. The sheath core 300 is formed by a PEEK tube 310 having a cavity 311 and a TIP head 320 coupled to the front end of the sheath core and matching the sheath tube 400.

The pusher 200, the sheath 400, and the rear end of the sheath core 300 are respectively coupled to the handle 500. The proximal end of the handle 500 connection is provided with a connector 510 that is coupled to the ablation power source. The proximal end of the guide wire 240 of the pusher 200 is electrically connected to the joint 510. The handle 500 is provided with independent motion mechanisms, which can realize mutually independent movements of the pusher 200, the sheath 400, and the sheath core 300.

In this embodiment, the interatrial shunt device 100, the pusher 200, the sheath core 300, the sheath 400, and the handle 500 are a complete system. The operation method of the interatrial shunt system of this embodiment is:
1) puncturing the atrial septum with a puncture mechanism; after the puncturing, sending the guide wire into the upper left pulmonary vein and withdrawing the puncture kit.
2) connecting the connector 510 at the proximal end of the handle to the RF power source (ablation power source) and pushing the opening device 100 that is pre-installed in the sheath along the guide wire into the body and arranging the front end of the sheath in the left atrium.
3) retracting the sheath tube 400 to completely unsheathe the left atrial positioning mechanism of the interatrial shunt device 100, and the left atrial positioning mechanism is fully opened; determining whether the left atrial positioning mechanism is fully opened by ultrasound or DSC; during the procedure, it is necessary to ensure that the distal end of the sheath is always in the left atrium; while making sure that there is no relative movement between the instruments pulling back the sheath 400 to make the left atrial positioning mechanism fitted against the atrial septum.
4) pulling back the sheath tube 400 to completely unsheathe the opening component 110 of the interatrial shunt device 100, determined by ultrasound or DSC, and the atrial septal tissue is opened by a small hole.
5) observing and making the electrode in good contact with the atrial septal tissue, then setting the heating parameters (such as power 30W, duration 120S), then starting the heating.
6) after the heating is stopped, pushing forward the sheath 400, making the right atrial positioning mechanism contracted to a smaller size and received into the sheath, and then pushing forward the sheath to completely recover the instruments to the sheath and withdrawing as a whole.

### Eighth Embodiment:

This embodiment is an improvement on the basis of embodiments first to seventh.

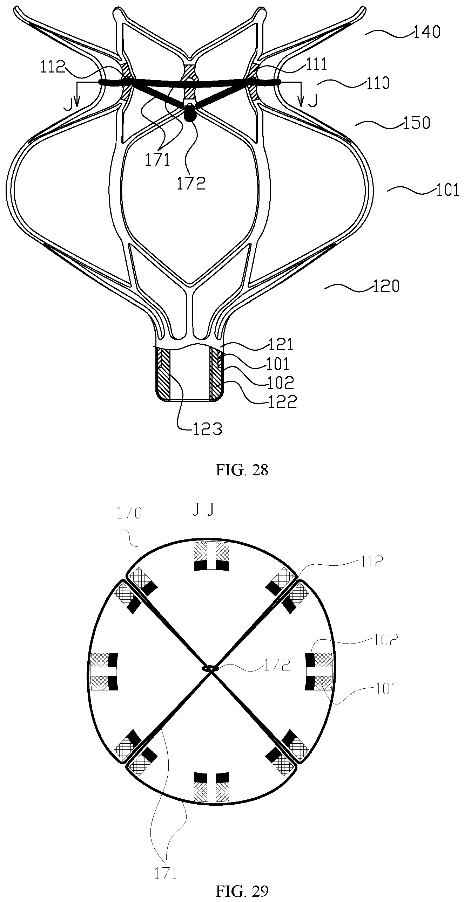

As shown in FIGS. 28-29, the interatrial shunt device 100 includes a tubular and radially expandable opening main body 101 and an opening component 110. The opening component 110 is provided with an ablation mechanism 111 electrically connected to the ablation power source and the control mechanism at least on the outer surface in a circumferential direction. The opening main body 101 and the opening component 110 are both struts and are formed by a plurality of struts cross-connecting, and the shape is spherical or spherical planar. In this embodiment, the positioning mechanism is formed by adopting and extending the structure conforming to the opening main body 101 and the opening component 110. At the distal end of the opening component 110 and the proximal end of the opening main body 101, two positioning mechanisms are respectively connected, i.e., a left atrial positioning mechanism 140 and a right atrial positioning echanism150, respectively.

The difference between the interatrial shunt device 100 and the embodiments first through seventh is that the opening component 110 is provided with an adjustment mechanism 170 for adjusting the radial diameter of the opening component 110. The radial adjustment mechanism 170 can have a variety of embodiments, as long as the radially constrained structure is implemented. And because the sheath is to be placed, the adjustment mechanism 170 needs to be radially contracted. Generally, a soft structure or a telescopic structure is used. The soft structure may be a control line. The adjustment mechanism 170 includes at least two control strings171. The two ends of the control string 171 pass through different positions of the opening component 110 in the circumferential direction and are converged to the center of the opening component 110 form a bundle. In this embodiment, the adjustment mechanism 170 includes four equal length control lines 171. The two ends of each control string 171 pass through the two adjacent control holes 112 from the outside of the opening component 110 towards inside, and each control hole has two wire ends. All of the wire ends are converged at the axis of the opening component 110 and merged to form a connecting ring 172 by knotting.

In another embodiment, the adjustment mechanism includes a control line. The control string simultaneously passes through different positions in the circumferential direction of the opening component and is fixed at both ends to limit the radial diameter of the opening component.

In another embodiment, the adjustment mechanism includes at least one control string. The control string passes through different positions in the circumferential direction of the opening component, and one end of each control string is fixed to the opening component or the distal end of the delivery system connected to the interatrial shunt device. The other end of the control string is connected to a control mechanism for controlling the implantation of the interatrial shunt device, so as to control the radial diameter of the opening component;

In another embodiment, the adjustment mechanism includes at least one control string. The control string passes through different positions in the circumferential direction of the opening component, and at least one end of each of the control strings passes through the delivery system, by manual operation, to control the radial diameter of the opening component.

If the adjustment mechanism adopts the telescopic structure, it can be an elastic ring, a coil spring, etc. The radial adjustment of the opening component 110 can be realized by adjusting the length or diameter of the elastic ring and the coil spring.

As shown in FIGS. 28-29, the interatrial shunt device 100 has an opening component 110 with a curved surface of the busbar recess in a fully released state. An ablation mechanism 111 is provided on the opening component 110. On the circumference of the smallest diameter of the opening component 110, four control holes 112 are evenly distributed.

At the distal end of the opening component 110 and the proximal end of the opening main body 101, a left atrial positioning mechanism 140 and a right atrial positioning mechanism 150 are respectively connected. The left atrial positioning mechanism 140 and the right atrial positioning mechanism 150 are tapered flanges. A conical retrieval department 120 is connected to the outer edge of the right atrial positioning mechanism 150, and the proximal end of the retrieval department 120 is contracted to the retrieval port 121, and the retrieval port 121 is connected to the metal nut 122.

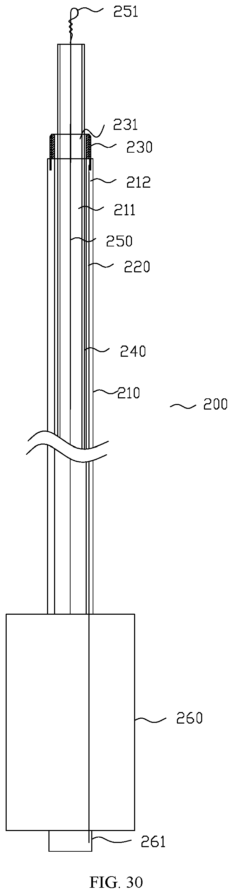

As shown in FIG. 30, the pusher 200 includes a single cavity tube 210, a guide wire 220, a distal bolt 230, a control tube 240, a traction line 250, and a control handle 260. The single cavity tube 210 includes a cavity 211, and the distal bolt 230 includes a cavity 231. The cavity 211 and the cavity 231 have the same shape and the coaxial line. The guide wire 220 is disposed in the tube wall 212 of the single cavity tube 210 and is electrically connected to the bolt 230 at the distal end. Except for the external threaded surface of the bolt 230, the other surfaces are coated with an insulating coating of PTFE.

The control tube 240 is disposed in the cavity 211 of the single cavity tube 210 and the cavity 231 of the bolt 230, and the traction line 250 is disposed in the cavity 241 of the control tube 240. The front end of the traction line 240 can be coupled to the connecting ring 172 of the adjustment mechanism 170. The proximal ends of the single cavity tube 210, the control tube 240, and the traction line 250 are connected to the control handles 260, respectively. The control handle 260 can control the mutually independent movements of the traction line 250 and the control tube 240. A proximal end of the control handle 260 is provided with a joint 261 that is coupled to the ablation power source. The proximal end of the guide wire 220 is electrically connected to the joint 261.

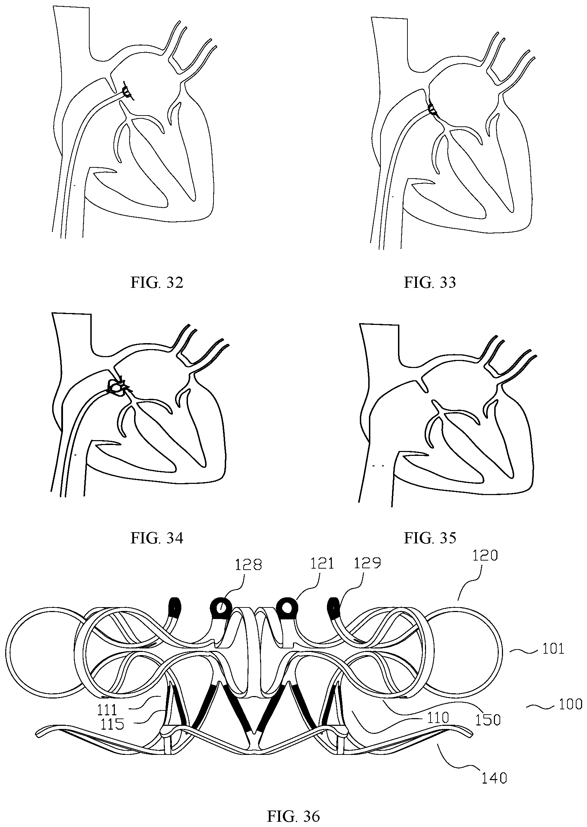

The opening device in this embodiment also needs to be used in combination with a loader, a sheath, an expander, an ablation power source, a power supply connection line, a neutral electrode plate, and the like. As shown in FIGS. 32-35, the interatrial shunt system of the present embodiment operates as follows:
1) puncturing the atrial septum with a puncture mechanism; after the puncturing, sending the guide wire into the upper left pulmonary vein and the puncture kit is removed; pushing the expander and the sheath along the guide wire into the left atrium, and the guide wire and the expander are removed.
2) making the pusher 200 pass through the proximal end of the loader, and the metal nut 123 at the proximal end of the interatrial shunt device 100 is connected to the distal bolt 230 of the pusher 200, and the front end of the traction line 240 is connected to the connecting ring 173 of the adjustment mechanism 170; the distal end of the control tube 240 and the control hole 112 of the interatrial shunt device 100 are adjusted by the control handle 260 to be on the same cross section perpendicular to the axis; then adjusting the traction line 250 to adjust the diameter of the opening component 110 of the interatrial shunt device 100 to about 3 mm; pulling back the pusher 200 to move the interatrial shunt device 100 into the loader.
3) connecting the distal end of the loader to the proximal end of the sheath; pushing forward the pusher 200 to deliver the interatrial shunt device 100 to the distal end of the sheath; then slowly pushing the pusher or retracting the sheath (ensuring that the distal end of the sheath is in the left atrium during the process), so that the left atrial positioning mechanism of the interatrial shunt device 100 is fully open, referring to FIG. 21; then making sure that there is no relative motion between the instruments and pulling the sheath back to attach the left atrial positioning mechanism to the atrial septum, see FIG. 22; then holding the shunt device and the pusher still and withdrawing the sheath, so that the opening component 110 and the right atrial positioning mechanism are fully opened and the right atrial positioning mechanism is attached to the atrial septum, see FIG. 23; the positions of the atrial septum and the ablation mechanism 111 can be observed by DSC to determine whether the attachment is complete.
4) adjusting the control handle 260 to make the size of the opening component 110 to the required size, and the size adjustment range is 2mm ∼ 14mm.
5) after confirming that the tissue at the opening is completely fitted with the ablation mechanism 111, connecting the proximal end of the pusher to the RF power source, and setting the heating parameters (such as power 20-80W, duration 10-50S), then starting the heating.
6) after the heating is stopped, the instruments can be recovered to the sheath and removed from the body, and the diameter of the opening is measured against the expectation, see FIG. 24.

### Ninth Embodiment:

This embodiment is an implantable embodiment.

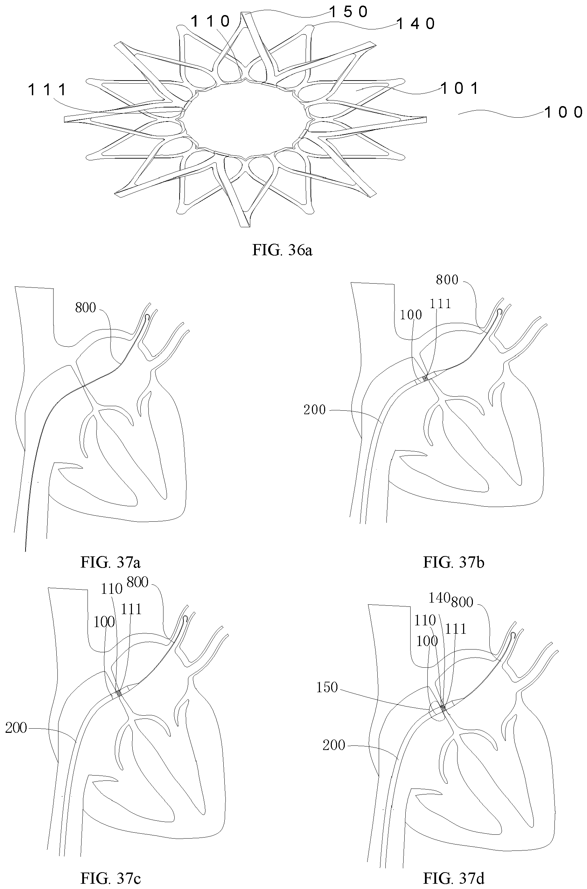

As shown in FIG. 36, the structure of this embodiment is basically the same as those of embodiments second through fifth except that the retrieval port 121 has a different structure.

The opening main body 101 is a disk-shaped structure, and the distal end and the proximal end of it are respectively connected with a left atrial positioning mechanism 140 and a right atrial positioning mechanism 150. The left atrial positioning mechanism 140 is a tapered flange, and the right atrial positioning mechanism 150 is a tapered flange.

A retrieval department 120 extending toward the proximal end and the axis is connected to the outer edge of the platform of the right atrial positioning mechanism 150. The retrieval department 120 is merged into four conductive retrieval ports 121 at the ends, and a circular retrieval hole 128 is disposed on the retrieval port 121. The four retrieval ports 121 are uniformly distributed on a circumference having a diameter larger than the diameter of the opening component 110. Except for the ablation mechanism 111 facing the outer surface 115 of the atrial septal tissue and the surface 129 of the retrieval port 121, the remaining surfaces are plated with a PTFE insulating coating 102. The surface 129 of the retrieval port 121 is used to electrically conduct with the ablation power source and the control mechanism.

The operation method of the interatrial shunt system of this embodiment is:
1) puncturing the atrial septum with a puncture mechanism; after the puncturing, the guide wire is sent into the upper left pulmonary vein and the puncture kit is removed; the expander and the sheath are pushed along the guide wire into the left atrium, and the guide wire and the expander are removed.
2) making the pusher pass from the proximal end of the loader, connecting the conductive retrieval port 121 of the interatrial shunt device 100 to the distal end of the pusher electrically, and withdrawing the pusher to move the interatrial shunt device 100 into the loader.
3) connecting the distal end of the loader to the proximal end of the sheath, pushing forward the pusher 200 to deliver the interatrial shunt device 100 to the distal end of the sheath; then pushing the pusher or retracting the sheath slowly, during the procedure, ensuring that the distal end of the sheath is in the left atrium, so that the left atrial positioning mechanism of the interatrial shunt device 100 is fully open; then making sure that there is no relative movement between the instruments and pulling back the sheath to attach the left atrial positioning mechanism to the atrial septum; holding the opening and the pusher still and withdrawing the sheath to allow the opening component 110, the right positioning mechanism, and the retrieval department 120 to fully open and the right positioning mechanism to fit closely on the atrial septum; the positions of the atrial septum and the ablation mechanism 111 can be observed by DSC to determine whether it is completely fitted.
4) after confirming that the tissue at the opening is completely fitted with the ablation mechanism 111, connecting the proximal end of the pusher to the RF power source, and setting the heating parameters (such as power 40W, duration 50S), then starting the heating.

After the heating is stopped, the instruments can be recovered to the sheath and removed from the body, or the opening device can be completely released as needed, and permanently implanted into the body. As shown in FIG. 36a, the opening main body 101 is provided with a channel in the axial direction. A shunt channel for atrial septal tissue can be formed when permanently implanted in the body.

### Tenth Embodiment:

This embodiment is an atrial septum opening method. Specifically, an interatrial shunt device is delivered to an atrial septal opening tissue in an interventional manner, and ablation is applied to the tissue in the opening cavity. The atrial septum tissue suffers an irreversible loss caused by ablation, so that the tissue at the opening is necrotic and loses most of the elasticity, making the shape of the opening stable and controllable.

The interatrial shunts method may applies ablating to the atrial septal opening tissue by one of heat, cold, light, electricity, gas, mechanical wave, electromagnetic wave, radioactive particle, chemical agent or any combination thereof. The ablation mechanism can cause protein denaturation of the atrial septal tissue cells in contact with it by a physical or chemical method, so that the opening structure is regularly stable and difficult to close.

Tissue cells are inactivated by heating or freezing the atrial septal tissue. In a specific embodiment, a radio frequency power source is used as an ablation source to inactivate atrial septal tissue cells by radio frequency ablation.

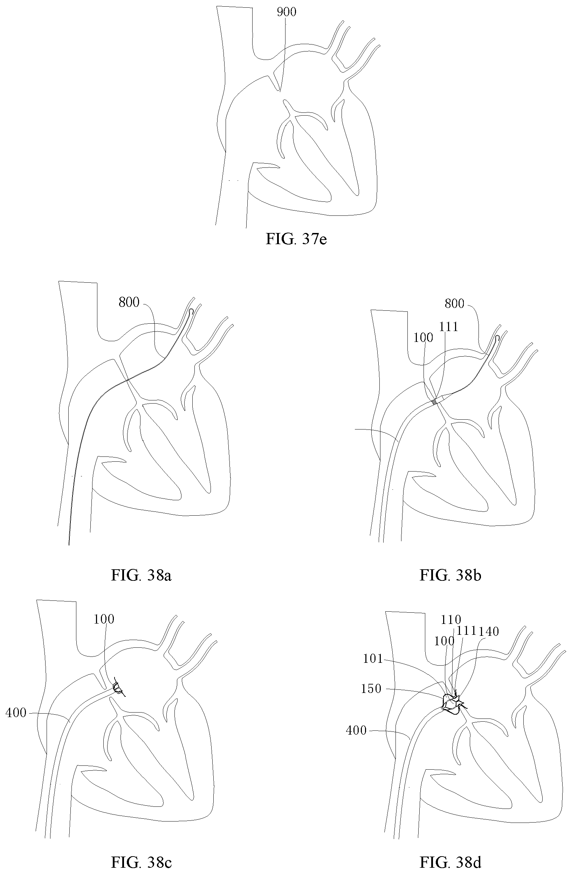

As shown in FIGS. 37a-37e, a specific interatrial shunts method of the present disclosure is illustrated. The interatrial shunt device 100 used in the present embodiment is a columnar structure overall, and a segment adjacent to the distal end is an opening component 110. The periphery of the opening component 110 is provided with an ablation mechanism 111. The ablation mechanism 111 can be electrically connected to the ablation power source by an interatrial shunt device control mechanism. The ablation mechanism can be stabilized at the ablation position by manually holding the control mechanism of the interatrial shunt device to fix the tubular ablation mechanism 111 by an operator. Or, the positioning mechanism 140 and/or the positioning mechanism 150 can be separately disposed on the both sides of the axial direction of the ablation mechanism 111 to prevent the ablation mechanism 111 from pulsating in response to the pulsation of the heart, thereby allowing it to be more stably fixed in the atrial septum where opening ablation is required. During the delivery process, the positioning mechanism 140 and the positioning mechanism 150 can be radially compressed in the sheath tube. After the sheath tube is retracted, the positioning mechanism 140 and the positioning mechanism 150 are deployed to abut both sides of the atrial septum, respectively. The ablation mechanism 111 is maintained at the opening component 900. The remaining structure can be seen and combined with other embodiments.

The device for ablation of atrial septal tissue opening in this embodiment includes an ablation mechanism in a columnar shape for annular ablation of intraluminal tissue and epidermal tissue at atrial septum opening. The annular ablation may ablate the tissue at the opening circumferentially and continuously for one or more rounds, or ablate a plurality of spaced points disposed circumferentially around the tissue at the opening for one round.

The atrial septum opening method of the embodiment includes the following steps:
1) puncturing the atrial septum through the femoral vein puncture, and retaining the guide wire 800 in the left atrium.
2) pushing the interatrial shunt device 100 along the guide wire and expanding the atrial septum; the interatrial shunt device 100 is integrally fixedly connected or detachably connected to the pusher 200 of the interatrial shunt device control mechanism.
3) under the observation of ultrasound or DSC, adjusting the position of the opening device, and making the annular ablation mechanism 111 of the opening device (may adopt a single annular electrode or a plurality of electrodes) in contact with the atrial septal tissue.
4) confirming that the annular ablation mechanism of the shunt device contacts the atrial septal tissue by means of impedance change and the like (when the ablation mechanism is attached to the atrial septal tissue, the impedance will increase significantly), and setting the ablation parameters (such as power 20-80W, duration 10-50S), discharging the ablation.
5) at the end of the ablation, the instrument is withdrawn from the body.

When the impedance becomes small, the end of the ablation can be determined.

### Eleventh Embodiment:

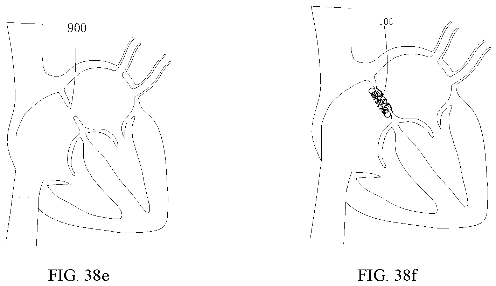

This embodiment is an interatrial shunts method, specifically an implantable embodiment, see FIGS. 38a-38e.

The interatrial shunt device 100 used in this embodiment includes an opening main body 101 at least a section of which is in a tubular structure in the axial direction. The shunt device 100 of one implementation in this embodiment is a radially expandable stent overall. The middle part of the opening main body 101 in the axial direction is an opening component 110. An ablation mechanism 111 is disposed on an outer circumference of the opening component. Both sides of the axis of the ablation mechanism 111 are respectively provided with a positioning mechanism 140 and a positioning mechanism 150. During the delivery process, the positioning mechanism 140 and the positioning mechanism 150 are radially compressed in the sheath 400. After retracting the sheath 400, the positioning mechanism is deployed, to respectively abut against both sides of the atrial septum, and the ablation mechanism is maintained at the opening component 900. The rest of the structure can be seen with reference to other embodiments.

In different implementations, the interatrial shunt device can also use a balloon. The fluid delivery device may be used as a balloon-sized adjustment mechanism to communicate with the balloon through a pipeline, and the fluid can be output to adjust the size of the balloon when in use. When the balloon is used, the middle portion of the balloon in the axial direction has a waist structure, i.e., the middle diameter becomes smaller and the diameters of both ends are larger. The balloon can dilate and expand at both ends to serve as a positioning mechanism.

The interatrial shunt device as described in FIGS. 1-9 can be used in the atrial septum opening method of this embodiment. The shunt device can include different forms of opening main body, such as an opening main body being a balloon, an elastic bracket or a combination of a balloon and an elastic bracket, that is capable of radially contracting and expanding. In the above-discussed opening main body, the opening component is optionally an elastic bracket capable of radially contracting and expanding, specifically a tubular structure or an annular structure formed by a wave structure, a mesh bracket, a rod bracket or a combination thereof.

The atrial septum opening method of the embodiment includes the following steps:
1) puncturing the femoral vein; puncturing the atrial septum (or performing first conventional interatrial shunt), and retaining the guide wire 900 in the left atrium.
2) pushing the sheath tube 400 along the guide wire 800 through and expanding the atrial septum.
3) under the observation of ultrasound or DSC, the sheath tube 400 is withdrawn and the interatrial shunt device 100 is adjusted to expand it to release it from the sheath 400, further expanding the atrial septal tissue; the ablation mechanism 111 is in contact with the atrial septal tissue.
   In various implementations, the radial diameter of the opening component can be controlled by an adjustment mechanism to obtain an adapted atrial septum opening size.
4) confirming that the ablation mechanism 111 is in contact with the atrial septal tissue, and discharging the ablation.
5) at the end of the ablation, the expandable interatrial shunt device 100 is withdrawn into the sheath 400 and then withdrawn from the body.

In another embodiment of the present disclosure, as shown in FIG. 38f, an interatrial shunt device 100 (optionally interatrial shunt device 100 of the ninth embodiment) is detachably attached to the interatrial shunt device control mechanism. After completion of the ablation, the interatrial shunt device 100 is released from the push rod of the device control mechanism, and the interatrial shunt device 100 can be left in the atrial septal tissue opening component to form a shunt channel. In this embodiment, the opening main body of the interatrial shunt device 100 is provided with a channel in the axial direction.

Although the respective embodiments have been described one by one, it shall be appreciated that the respective embodiments will not be isolated. Those skilled in the art can apparently appreciate upon reading the disclosure of the application that the respective technical features involved in the respective embodiments can be combined arbitrarily between the respective embodiments as long as they have no collision with each other.

The foregoing implementations are merely specific embodiments of the present disclosure, and are not intended to limit the protection scope of the present disclosure. It should be noted that any variation or replacement readily figured out by persons skilled in the art within the technical scope disclosed in the present disclosure shall all fall into the protection scope of the present disclosure. Therefore, the protection scope of the present disclosure shall be subject to the protection scope of the claims.

## Claims

1. An interatrial shunts device, comprising an opening main body,
the opening main body comprises an opening component,
wherein the opening component penetrates through an atrial septum and expands an atrial septal tissue to form an atrial septal opening, an ablation mechanism capable of damaging tissue activity of the atrial septal opening is disposed where the opening component contacts the atrial septal tissue.

2. The interatrial shunts device of claim 1, further comprising an extension component connected with the opening main body, wherein the extension component is configured for compensating or preventing a deviation of the ablation mechanism from the atrial septal opening.

3. The interatrial shunts device of claim 1, wherein the ablation mechanism ablates an atrial septal opening tissue by any or a combination of heating, cooling, lighting, electrifying, gas, mechanical waves, electromagnetic waves, radioactive particles, and chemicals.

4. The interatrial shunts device of claim 1, wherein the ablation mechanism increases or decreases a local temperature of the atrial septal tissue in contact with the ablation mechanism.

5. The interatrial shunts device of claim 4, wherein the ablation mechanism is a radio frequency ablation mechanism, the radio frequency ablation mechanism is electrically conductive with an ablation power source during operation.

6. The interatrial shunts device of claim 1, wherein the opening main body comprises at least an opening component in a columnar structure.

7. The interatrial shunts device of claim 1, wherein a radial diameter of the opening component is uniform.

8. The interatrial shunts device of claim 1, wherein the opening main body comprises at least an opening component in a tubular structure.

9. The interatrial shunts device of claim 8, wherein the opening component is provided with a compressed state in which a radial diameter is adjustable and an expanded state.

10. The interatrial shunts device of claim 8, wherein the opening main body is a balloon capable of radially contracting and expanding, an elastic bracket capable of radially contracting and expanding, or a combination of the balloon and the elastic bracket.

11. The interatrial shunts device of claim 2, wherein after the opening main body penetrates through the atrial septum, the extension component is divided by the atrial septum into a first part of the extension component on one side of the atrial septum and a second part of the extension component on the other side of the atrial septum,
wherein the ablation mechanism extends along an axial direction of the opening main body together with the opening component, when the opening main body penetrates through the atrial septum the ablation mechanism extends to one or two sides outside the atrial septal opening, a part of the ablation mechanism located outside the atrial septal opening being the first part of the extension component,
wherein the second part of the extension component is configured as:
the opening main body is connected with at least one positioning mechanism, when the opening main body penetrates through the atrial septum the positioning mechanism abuts against an outer periphery of the atrial septal opening on a corresponding side of the atrial septum.

12. The interatrial shunts device of claim 11, wherein the opening main body is disposed on one side of the atrial septum or on both sides of the atrial septum when the opening main body penetrates through the atrial septum.

13. The interatrial shunts device of claim 11, wherein the positioning mechanism is disposed on one or both sides of the ablation mechanism.

14. The interatrial shunts device of claim 11, wherein the positioning mechanism is provided with a positioning surface, a positioning line, or a positioning point abutting against the atrial septum, and the positioning surface is a plane, a cone, an arc, or a combination of the plane, the cone, and the arc.

15. The interatrial shunts device of claim 11, wherein the positioning mechanism is coupled to a thrombosis capturing mechanism.

16. The interatrial shunts device of claim 15, wherein the thrombosis capturing mechanism is a cage-shape structure.

17. The interatrial shunts device of claim 11, wherein the positioning mechanism is provided with at least one radiopaque marker.

18. The interatrial shunts device of claim 5, wherein the ablation mechanism is a metal exposed on an outer surface of the opening component or a metal electrode fixed on the outer surface of the opening component.

19. The interatrial shunts device of claim 18, wherein a proximal end of the opening main body is provided with a retrial department, and the retrial department is provided with a connector for connecting with a control mechanism of the opening main body, the connector is electrically conductive with the ablation mechanism.

20. The interatrial shunts device of claim 19, wherein the outer surfaces of the opening main body and the opening component are insulative except where the ablation mechanism contacts the atrial septal tissue.

21. The interatrial shunts device of claim 18, wherein the ablation mechanism is provided with a connecting part for connecting a control mechanism of the opening main body, the connecting part being electrically conductive with the ablation mechanism.

22. The interatrial shunts device of claim 21, wherein an insulator is disposed between the ablation mechanism and the opening component to prevent electrical conduction between the ablation mechanism and the opening component.

23. The interatrial shunts device of claim 9, wherein the opening component is provided with an adjustment mechanism that adjusts the radial diameter of the opening component.

24. The interatrial shunts device of claim 23, wherein the adjustment mechanism comprises at least one control line, the control line simultaneously passing through different positions of the opening component circumferentially, a dimension of the opening component being adjusted by controlling a length of a line crossing the circumferential direction of the opening component.

25. The interatrial shunts device of claim 23, wherein the adjustment mechanism comprises at least two control lines, each of the at least two control lines respectively passing through a different position of the opening component in the circumferential direction and converging toward a center of the opening component into a bundle, and fixed to limit the radial diameter of the opening component.

26. The interatrial shunts device of claim 23, wherein the adjustment mechanism comprises a fluid delivery device in communication with a balloon.

27. The interatrial shunts device of claim 5, wherein the ablation mechanism is coupled to a temperature sensor in contact with the atrial septal tissue, the temperature sensor being electrically coupled to the ablation power source.

28. The interatrial shunts device of claim 1, wherein the ablation mechanism is provided with at least one development point.

29. An interatrial shunts system, comprising the interatrial shunts device, the control mechanism of the opening device, and the ablative power source according to any of claims 1-28, wherein the ablation power source is electrically coupled to the ablation mechanism via the control mechanism of the opening device.

30. The interatrial shunts system of claim 29, wherein the opening device is integrally fixedly connected to or detachably fixedly connected to the control mechanism of the opening device.

31. The interatrial shunts system of claim 29, wherein the control mechanism of the opening device comprises a pusher of the opening device.

32. The interatrial shunts system of claim 29, wherein the control mechanism of the opening device comprises a pusher of the opening device, and an outer sheath assembly that carries the pusher and the opening device.

33. The interatrial shunts system of claim 29, wherein the control mechanism of the opening device comprises a pusher of the opening device, an outer sheath assembly that carries the pusher and the opening device, and a sheath core assembly inserted into the pusher.

34. The interatrial shunts system of claim 29, wherein the control mechanism of the opening device comprises an adjustment assembly that controls the radial diameter of the opening component.

35. The interatrial shunts system of claim 34, wherein the adjustment assembly comprises a traction line and a control tube.

36. The interatrial shunts system of claim 34, wherein the adjustment assembly comprises a fluid delivery device.

37. A method of operating an interatrial shunts system, comprising:
puncturing an atrial septum;
using the interatrial shunts device according to any of claims 1-28 and delivering the interatrial shunts device to an atrial septal tissue opening by the control mechanism of the opening device;
releasing the interatrial shunts device and forming a shunt channel on the atrial septal tissue by the opening component of the interatrial shunts device;
determining and confirming that a tissue at the atrial septal opening is completely conformed to the ablation mechanism of the interatrial shunts device, and launching the ablation;
after the ablation stops, withdrawing the interatrial shunts device.

38. The method of operating the interatrial shunts system according to claim 37, wherein in the step of delivering the interatrial shunts device to the atrial septal tissue opening by the control mechanism of the opening device, the control mechanism of the opening device comprises an outer sheath, and the interatrial shunts device is loaded into the outer sheath and then transported to the atrial septal tissue opening.

39. The method of operating the interatrial shunts system according to claim 37, wherein in the step of releasing the interatrial shunts device and forming a shunt channel on the atrial septal tissue by the opening component of the interatrial shunts device, the interatrial shunts device is radially expandable to further expand the tissue.

40. The method of operating the interatrial shunts system according to claim 37, wherein in the step of releasing the interatrial shunts device and forming a shunt channel on the atrial septal tissue by the opening component of the interatrial shunts device, the radial diameter of the interatrial shunts device is adjustable.

41. The method of operating the interatrial shunts system according to claim 37, wherein the interatrial shunts device comprises a positioning mechanism, the positioning mechanism stabilizing the opening device at the atrial septal opening before the launching the ablation.

42. The method of operating the interatrial shunts system according to claim 37, wherein in the step of determining and confirming that the tissue at the atrial septal opening is completely conformed to the ablation mechanism of the interatrial shunts device, determining whether the opening device is conformed to the atrial septal tissue by monitoring an impedance change.

43. A method of forming an atrial septum opening, comprising:
puncturing an atrial septum to form an atrial septal opening, wherein an interatrial shunts device is delivered into the atrial septum opening, and a tissue at the atrial septum opening is ablated to cause the tissue lose activity.

44. The method of claim 43, wherein the tissue at the atrial septal opening is a tissue within and around a cavity of the atrial septal opening.

45. The method of claim 43, wherein the interatrial shunts device comprises an opening main body, the opening main body is provided with an opening component for penetrating within the atrial septum opening and expanding an atrial septal opening tissue, the opening component is provided with an ablation mechanism for ablating the atrial septal opening tissue.

46. The method of claim 45, wherein the ablation performed on the atrial septum opening tissue is radio frequency ablation.

47. The method of claim 46, wherein the ablation mechanism is electrically coupled to an ablation power source during operation.

48. The method of claim 47, wherein the ablation mechanism of the interatrial shunts device is electrified after determining and confirming that a contact position of the ablation mechanism with the atrial septum is appropriate.

49. The method of claim 48, wherein the step of determining and confirming that the contact position of the ablation mechanism with the atrial septum is appropriate is achieved by monitoring an impedance change.

50. The method of claim 47, wherein the interatrial shunts device is provided with an extension component connected with the opening main body, the extension component is configured for compensating or preventing a deviation of the ablation mechanism from the atrial septal opening,
wherein after the opening main body penetrates through the atrial septum, the extension component is divided by the atrial septum into a first part of the extension component on one side of the atrial septum and a second part of the extension component on the other side of the atrial septum,
wherein the ablation mechanism extends along an axial direction of the opening main body together with the opening component, when the opening main body penetrates through the atrial septum the ablation mechanism extends to one or two sides outside the atrial septal opening, a part of the ablation mechanism located outside the atrial septal opening being the first part of the extension component,
wherein the second part of the extension component is configured as:
the opening main body is connected with at least one positioning mechanism, when the opening main body penetrates through the atrial septum the positioning mechanism abuts against an outer periphery of the atrial septal opening on a corresponding side of the atrial septum.

51. The method of claim 50, wherein a positioning mechanism is disposed in the interatrial shunts device, the ablation mechanism of the interatrial shunts device is electrified after determining and confirming that a contact position of the ablation mechanism with the atrial septum is appropriate via modifying and maintaining the contact between the ablation mechanism and the atrial septum by the positioning mechanism.

52. The method of claim 51, wherein the opening main body is coupled to at least one positioning mechanism, the positioning mechanism being disposed on one or both sides of the ablation mechanism.

53. The method of claim 52, wherein at least one of the positioning mechanisms has a radial diameter that is greater than a radial diameter of the ablation mechanism.

54. The method of claim 43, wherein the interatrial shunts device comprises an opening main body, the opening main body comprising at least an opening component in a columnar structure.

55. The method of claim 54, wherein the radial diameter of the opening component is unchanged.

56. The method of claim 43, wherein the interatrial shunts device comprises an opening main body, the opening main body comprising at least an opening component in a tubular structure.

57. The method of claim 56, wherein the opening component is provided with a compressed state in which a radial diameter is adjustable and an expanded state.

58. The method of claim 56, wherein the opening main body is a balloon capable of radially contracting and expanding, an elastic bracket capable of radially contracting and expanding, or a combination of the balloon and the elastic bracket.

59. The method of claim 56, wherein the opening component is an elastic bracket capable of radially contracting and expanding in a wave structure, a mesh bracket, a rod bracket or a combination thereof.

60. The method of claim 43, wherein the interatrial shunts device is delivered to the atrial septum opening via a control mechanism of the interatrial shunts device.

61. The method of claim 60, wherein the control mechanism is fixedly connected or detachably fixedly connected to the interatrial shunts device.

62. The method of claim 60, wherein the control mechanism withdraws the interatrial shunts device after the ablation is completed.

63. The method of claim 60, wherein the control mechanism comprises an outer sheath, the control mechanism enabling a complete recovery of the interatrial shunts device into the sheath after completion of the ablation, and an overall withdraw.

64. The method of claim 60, wherein upon completion of the ablation, the control mechanism is released from the interatrial shunts device, the control mechanism is withdrawn, and the interatrial shunts device is disposed at the atrial septal tissue opening.

65. The method of claim 64, wherein the interatrial shunts device is provided with a channel in an axial direction.
